# EUROPEAN PATENT APPLICATION

(11) **EP 4 744 681 A1**
(43) Date of publication of application: **20.05.2026**
(21) Application number: 25849688.4
(22) Date of filing: 06.08.2025
(51) Int. Cl.: A61K 47/68, A61P 35/00, C07K 16/28

(54) **TARGET CELL-TARGETING COMPLEX AND USE THEREOF**

(30) Priority: 16.08.2024 WO PCT/CN2024/112583; 27.12.2024 CN 202411955314
(71) Applicant: Shanghai Bao Pharmaceuticals Co., Ltd., Shanghai 201908 (CN)
(72) Inventor: WANG, Zheng, Shanghai 201908 (CN); LIU, Yanjun, Shanghai 201908 (CN); XU, Yunxia, Shanghai 201908 (CN); LI, He, Shanghai 201908 (CN); LV, Baojie, Shanghai 201908 (CN)
(74) Representative: Bayramoglu et al.
(86) International application number: PCT/CN2025/113100
(87) International publication number: WO 2026/037175

(57) **Abstract**

The present disclosure discloses a target cell-targeting complex and a use thereof. The complex includes: (1) a first molecule which is a protein containing an Fc fragment moiety; and (2) a second molecule containing an enzyme and a fragment that can non-covalently binding to the first molecule to form a stable structure. The complex retains the activity of each portion and can exert the effects of targeting proteins and enzymes by administering the targeting complex to a subject.

## Description

### PRIORITY CLAIM

This application claims priority to International Application No. PCT/CN2024/112583, filed on August 16, 2024, and to Chinese Patent Application No. CN2024119553147, filed on December 27, 2024, which are incorporated herein by reference in their entirety.

### TECHNICAL FIELD

The present disclosure relates to the field of biomedicine, in particular to target cell-targeting complex and a use of the complex.

### BACKGROUND TECHNOLOGY

The rise of immunotherapy has brought a paradigm shift for difficult-to-treat tumors. Immune cells integrate signals from activating and inhibitory receptors to determine their response to challenging targets. Tumors often hinder immune cell recognition by overexpressing ligands for inhibitory receptors. This discovery has led to the emergence of new therapeutic strategies aimed at blocking inhibitory immune cell signaling, such as clinically approved T cell checkpoint inhibitors targeting PD-1 and CTLA-4. Ongoing preclinical research focuses on combining therapies that target multiple immune pathways. For example, the combination of antibodies targeting PD-1/PD-L1 with antibodies targeting other T cell checkpoint inhibitors has shown improved anti-tumor activity in syngeneic tumor models. Complementing these interventions are therapies targeting innate immune cells, particularly natural killer (NK) cells, macrophages, and dendritic cells.

Cancer immunotherapy using immune checkpoint inhibitors, including antibodies that block the PD-1/PD-L1 pathway, has improved outcomes for many cancer patients. However, despite the progress achieved to date, many patients do not respond to currently available immune checkpoint inhibitors.

There is usually an abnormal increase or abnormal expression of cell surface molecules or extracellular matrix molecules such as sialic acid, hyaluronic acid, heparin, collagen, and chondroitin on or around tumor cells. For example, hypersialylation is frequently observed in tumor tissues compared to corresponding normal tissues. The levels of total sialic acid in serum or glycolipid-bound sialic acid are significantly increased in patients with various cancers, including ovarian cancer, leukemia, colorectal cancer, and breast cancer. Hypersialylation leads to accelerated cancer progression and poor prognosis. Increased sialic acid in tumor cells is primarily caused by abnormal metabolism and abnormal expression of sialyltransferases/sialidases. It has been reported that sialic acid metabolism is upregulated in highly metastatic breast tumors, and the knockout of the CMAS gene, a key node in sialic acid metabolism, can inhibit sialic acid activation and reduce the formation of lung metastases in vivo. On the other hand, abnormal expression of sialyltransferases and sialidases accelerates and maintains the hypersialylated state of glycoconjugates. Sialylation of tumor cells further promotes immune escape, enhances tumor proliferation and metastasis, promotes tumor angiogenesis, and aids resistance to apoptosis and cancer therapy.

The abnormal increase or abnormal expression of cell surface molecules or extracellular matrix molecules not only occurs in tumor cells, but also widely exists in various diseases and corresponding cell types. For example, overexpression of hyaluronic acid leads to joint swelling and pain, HIV virus relies on the sialic acid metabolic pathway of host cells for envelope formation, and excessive secretion of collagen and hyaluronic acid in hepatitis leads to fibrotic diseases. Although these diseases can usually be treated with antibodies, the abnormal increase or abnormal expression of these cell surface molecules or extracellular matrix molecules greatly reduces the efficacy of antibodies.

Therefore, targeted drugs such as antibodies alone are not sufficient to achieve therapeutic effects, and it may also be necessary to simultaneously remove the "camouflage" on the surface and matrix of target cells. A new therapeutic strategy is urgently needed that enables immunotherapy to break through the limitations of barriers formed by cell surface molecules or extracellular matrix molecules, thereby truly restoring and amplifying the immune response.

### CONTENT OF THE INVENTION

An object of the present disclosure is to provide a target cell-targeting complex and a use of the complex.

A first aspect of the present disclosure provides a targeting complex including: a first molecule including an Fc fragment moiety and a target-binding fragment moiety; and a second molecule including a first moiety that is an enzyme and a second moiety capable of non-covalently binding to the first molecule. In an optional embodiment, the first molecule is a targeting protein including an Fc fragment moiety and a target-binding fragment moiety, and the second molecule is a fusion protein.

In some embodiments, the first molecule includes an antibody or a fusion protein including an Fc fragment. In an optional embodiment, the antibody is selected from an anti-Her2 antibody, an anti-PD-1 antibody, or an anti-PD-L1 antibody.

In some embodiments, the non-covalent binding is a protein-protein interaction. In an optional embodiment, the protein-protein interaction is selected from antigen-antibody binding, Protein A/Protein G-antibody Fc binding, or antibody Fc receptor (FcR)-antibody Fc binding.

In some embodiments, the second molecule retains enzymatic activity and/or the first molecule retains targeting activity. In an optional embodiment, after the targeting complex binds to a target molecule, the second molecule is capable of acting on a substrate molecule of the enzyme around the target molecule.

In some embodiments, the enzyme is an extracellular molecule-modifying enzyme.

In an optional embodiment, the enzyme is selected from a hyaluronidase, a sialidase, an N-glycosidase, an O-glycosidase, a collagenase, a heparinase, a chondrosulphatase , or a chondroitinase.

In an optional embodiment, the sialidase is selected from Salmonella typhimurium sialidase, Vibrio cholerae sialidase, human neuraminidase 1, human neuraminidase 2, human neuraminidase 3, or human neuraminidase 4.

In an optional embodiment, the N-glycosidase is selected from Endo H, PNGase F, PNGase A, Endo S, Endo S2, Endo D, Endo F2, or Endo F3.

In an optional embodiment, the O-glycosidase is selected from Endo-O-Glycosidase or Exo-O-Glycosidase.

In an optional embodiment, the hyaluronidase is selected from HYAL1, HYAL2, HYAL3, HYAL4, HYALP1, or PH20/SPAM1.

In an optional embodiment, the chondroitinase is selected from chondroitinase C, chondroitinase B, hyaluronidase ACIII, chondroitinase ACII, chondroitinase AC, or chondroitinase ABC.

In an optional embodiment, the heparinase is selected from heparinase I, heparinase II, or heparinase III.

In an optional embodiment, the collagenase is selected from collagenase 1, collagenase 2, collagenase 3, or serine protease Cathepsin L.

In some embodiments, the second moiety of the second molecule is capable of non-covalently binding to a constant region of the first molecule. In an optional embodiment, the second moiety of the second molecule is capable of non-covalently binding to the Fc fragment moiety of the first molecule. In an optional embodiment, an affinity, expressed as KD, of the second moiety of the second molecule to the Fc fragment moiety of the first molecule is 1 × 10⁻⁷ M to 1 × 10⁻¹² M. In an optional embodiment, an affinity KD of the second moiety of the second molecule to the Fc fragment moiety of the first molecule is 1 × 10⁻⁸ M to 1 × 10⁻¹¹ M. In an optional embodiment, the second moiety of the second molecule is selected from an antibody or an antigen-binding fragment thereof (for example, VHH, scFv, Fab or Fab2) capable of binding to a constant region fragment of an antibody, or selected from Protein A or an Fc-binding fragment thereof, affibody or an Fc-binding fragment thereof, Protein G or an Fc-binding fragment thereof, or Fc receptor (FcR) or an Fc-binding fragment thereof capable of binding to an Fc region of an antibody, or the second moiety of the second molecule is an Fc-binding peptide.

In some embodiments, the second moiety of the second molecule is located at an N-terminus or a C-terminus of the first moiety, and preferably, the second moiety of the second molecule is located at the N-terminus of the first moiety.

In an optional embodiment, the enzyme and the second moiety of the second molecule are connected via a peptide bond or a linker. In an optional embodiment, the linker is a peptide linker.

In some embodiments, the second moiety of the second molecule is a wild-type Z33 or a polypeptide having an amino acid mutation relative to the wild-type Z33.

In some embodiments, a position of the amino acid mutation includes at least one of positions 1, 4 and 29, and the wild-type Z33 has an amino acid sequence shown in SEQ ID NO: 1.

In some embodiments, the polypeptide having an amino acid mutation relative to the wild-type Z33 has an amino acid substitution relative to the wild-type Z33. In an optional embodiment, the substituted amino acid at the position 1 is selected from F or L. In an optional embodiment, the substituted amino acid at the position 4 is selected from Q, F, E, R, or L. In an optional embodiment, the substituted amino acid at the position 29 is selected from S, Q, R, or K.

In some embodiments, the amino acid sequence of the polypeptide having an amino acid mutation relative to the wild-type Z33 further has at least 50%, at least 60%, at least 70%, at least 80%, at least 85%, at least 90%, at least 92%, at least 95%, or at least 97% identity to the amino acid sequence shown in SEQ ID NO: 1.

In some embodiments, the polypeptide having an amino acid mutation relative to the wild-type Z33 includes the following amino acid sequence: 
X1X2X3X4X5X6X7X8X9X10X11X12X13X14X15X16X17X18X19X20X21X22X23X24X25X26X
27X28X29X30X31X32X33,
 where
X₁ is selected from F or L,
X₄ is selected from Q, F, E, R, or L,
X₂₉ is selected from S, Q, R, or K, further
X₂ is selected from N, E, or S,
X₃ is selected from M, K, or E,
X₅ is selected from Q or C,
X₆ is selected from Q, W, L, K, or A,
X₇ is selected from R, L, K, N, A, D, or C,
X₈ is selected from R or A,
X₉ is selected from F, M, or A,
X₁₀ is selected from Y, A, or S,
X₁₁ is selected from E, A, D, or S,
X₁₂ is selected from A, I, T, or E,
X₁₃ is selected from L, A, G, or K,
X₁₄ is selected from H, A, V, F, W, or G,
X₁₅ is selected from D, V, C, N, or L,
X₁₆ is selected from P, L, or A,
X₁₇ is selected from N, L, T, or G,
X₁₈ is selected from L, M, or V,
X₁₉ is selected from N, G, or T,
X₂₀ is selected from E, K, A, or Q,
X₂₁ is selected from E or Q,
X₂₂ is Q,
X₂₃ is R,
X₂₄ is selected from N, W, A, or K,
X₂₅ is selected from A or G,
X₂₆ is selected from K, R, or F,
X₂₇ is I,
X₂₈ is selected from K, C, Q, or R,
X₃₀ is selected from I, F, or L,
X₃₁ is selected from R, M, or K,
X₃₂ is selected from D, N, K, or E, and
X₃₃ is selected from D, N, or E.

In some embodiments, X₇ is R, X₉ is F, X₁₀ is Y, X₁₃ is L, X₂₃ is R, and/or X₃₀ is I.

In some embodiments, X₁₁ is E or D, X₁₂ is A or T, X₁₄ is H or W, X₁₆ is P, A, or L, X₁₇ is N, L, or T, X₁₈ is N or M, and/or X₂₃ is R.

In some embodiments, X₁ is L, X₄ is L, and/or X₂₉ is Q, K, or R.

In some embodiments, the hyaluronidase has an amino acid sequence shown in any one of SEQ ID NOs. 6, 7, or 65 to 71, the sialidase has an amino acid sequence shown in any one of SEQ ID NOs. 4, 5, or 55 to 60, the collagenase has an amino acid sequence shown in SEQ ID NO. 8, the heparinase has an amino acid sequence shown in SEQ ID NO. 9, the chondrosulphatase has an amino acid sequence shown in SEQ ID NO. 10, and the chondroitinase has an amino acid sequence shown in SEQ ID NO. 11.

In some embodiments, the second moiety of the second molecule is an antibody or an antigen-binding fragment thereof including heavy chain variable regions CDR1 to CDR3, and amino acid sequences of the heavy chain variable regions CDR1 to CDR3 respectively include SEQ ID NOs. 31 to 33 according to the Kabat numbering scheme.

In an optional embodiment, the second moiety of the molecule includes a polypeptide as shown in any one of SEQ ID NOs. 12 to 18 and SEQ ID NOs. 34 to 39, or an Fc-binding fragment thereof.

In an optional embodiment, the amino acid sequence of the second moiety of the second molecule is selected from a polypeptide as shown in any one of SEQ ID NOs. 34 to 39, or an Fc-binding fragment thereof.

In an optional embodiment, an amino acid sequence of the second moiety of the second molecule is SEQ ID NO. 34, 36, 38, or 39.

In some embodiments, the fusion protein serving as the second molecule is selected from a polypeptide having an amino acid sequence shown in any one of SEQ ID NOs. 19 to 30 or SEQ ID NOs. 61 to 64.

A second aspect of the present disclosure provides a molecule including: a first moiety that is an enzyme; and a second moiety capable of non-covalently binding to a constant region of an antibody, in an optional embodiment, the molecule is a fusion protein.

In some embodiments, the non-covalent binding is a protein-protein interaction, and in an optional embodiment, the protein-protein interaction is selected from antigen-antibody binding, Protein A/Protein G-antibody Fc binding, or antibody Fc receptor-antibody Fc binding.

In some embodiments, an affinity, expressed as KD, of the second moiety to the constant region of the antibody is 1 × 10⁻⁷ M to 1 × 10⁻¹² M. In some embodiments, an affinity KD of the second moiety to the constant region of the antibody is 1 × 10⁻⁸ M to 1 × 10⁻¹¹ M.

In some embodiments, the molecule retains enzymatic activity. In an optional embodiment, after binding to a first molecule, the molecule is capable of modifying a substrate molecule of the enzyme around a target of the first molecule, and the first molecule includes an Fc fragment moiety and a target-binding fragment moiety. In an optional embodiment, the first molecule includes an antibody or a fusion protein including an Fc fragment.

In some embodiments, the enzyme is an extracellular molecule-modifying enzyme.

In an optional embodiment, the enzyme is selected from a hyaluronidase, a sialidase, an N-glycosidase, an O-glycosidase, a collagenase, a heparinase, a chondrosulphatase, or a chondroitinase.

In an optional embodiment, the sialidase is selected from Salmonella typhimurium sialidase, Vibrio cholerae sialidase, human neuraminidase 1, human neuraminidase 2, human neuraminidase 3, or human neuraminidase 4.

In an optional embodiment, the N-glycosidase is selected from Endo H, PNGase F, PNGase A, Endo S, Endo S2, Endo D, Endo F2, or Endo F3.

In an optional embodiment, the O-glycosidase is selected from Endo-O-Glycosidase or Exo-O-Glycosidase.

In an optional embodiment, the hyaluronidase is selected from HYAL1, HYAL2, HYAL3, HYAL4, HYALP1, or PH20/SPAM1.

In an optional embodiment, the chondroitinase is selected from chondroitinase C, chondroitinase B, hyaluronidase ACIII, chondroitinase ACII, chondroitinase AC, or chondroitinase ABC.

In an optional embodiment, the heparinase is selected from heparinase I, heparinase II, or heparinase III.

In an optional embodiment, the collagenase is selected from collagenase 1, collagenase 2, collagenase 3, or serine protease Cathepsin L.

In some embodiments, the second moiety of the molecule is capable of non-covalently binding to a constant region of the first molecule.

In an optional embodiment, the second moiety of the molecule is capable of non-covalently binding to an Fc region of the first molecule. In an optional embodiment, the second moiety of the molecule is selected from an antibody or an antigen-binding fragment thereof capable of binding to a constant region fragment of an antibody (for example, VHH, scFv, Fab or Fab2), or selected from Protein A or an Fc-binding fragment thereof, Protein G or an Fc-binding fragment thereof, or FcR or an Fc-binding fragment thereof capable of binding to an Fc region of an antibody, or the second moiety of the molecule is an Fc-binding peptide.

In some embodiments, the second moiety of the molecule is the second moiety of the second molecule in the targeting complex as described above.

In some embodiments, the second moiety of the molecule is located at an N-terminus or a C-terminus of the first moiety, and preferably, the second moiety of the molecule is located at the N-terminus of the first moiety.

In some embodiments, the second moiety of the molecule is a wild-type Z33 or a polypeptide having an amino acid mutation relative to the wild-type Z33. In some embodiments, a site of the amino acid mutation includes at least one of positions 1, 4 and 29, and the wild-type Z33 has an amino acid sequence shown in SEQ ID NO: 1. In some embodiments, the polypeptide having an amino acid mutation relative to the wild-type Z33 has an amino acid substitution relative to the wild-type Z33. In an optional embodiment, the substituted amino acid at the position 1 is selected from F or L. In an optional embodiment, the substituted amino acid at the position 4 is selected from Q, F, E, R, or L. In an optional embodiment, the substituted amino acid at the position 29 is selected from S, Q, R, or K.

In some embodiments, the amino acid sequence of the polypeptide having an amino acid mutation relative to the wild-type Z33 further has at least 50%, at least 60%, at least 70%, at least 80%, at least 85%, at least 90%, at least 92%, at least 95%, or at least 97% identity to the amino acid sequence shown in SEQ ID NO: 1.

In some embodiments, the polypeptide having an amino acid mutation relative to the wild-type Z33 includes the following amino acid sequence: 
X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃X₂₄X₂₅X₂₆X
₂₇X₂₈X₂₉X₃₀X₃₁X₃₂X₃₃,
 where
X₁ is selected from F or L,
X₄ is selected from Q, F, E, R, or L,
X₂₉ is selected from S, Q, R, or K,
X₂ is selected from N, E, or S,
X₃ is selected from M, K, or E,
X₅ is selected from Q or C,
X₆ is selected from Q, W, L, K, or A,
X₇ is selected from R, L, K, N, A, D, or C,
X₈ is selected from R or A,
X₉ is selected from F, M, or A,
X₁₀ is selected from Y, A, or S,
X₁₁ is selected from E, A, D, or S,
X₁₂ is selected from A, I, T, or E,
X₁₃ is selected from L, A, G, or K,
X₁₄ is selected from H, A, V, F, W, or G,
X₁₅ is selected from D, V, C, N, or L,
X₁₆ is selected from P, L, or A,
X₁₇ is selected from N, L, T, or G,
X₁₈ is selected from L, M, or V,
X₁₉ is selected from N, G, or T,
X₂₀ is selected from E, K, A, or Q,
X₂₁ is selected from E or Q,
X₂₂ is Q,
X₂₃ is R,
X₂₄ is selected from N, W, A, or K,
X₂₅ is selected from A or G,
X₂₆ is selected from K, R, or F,
X₂₇ is I,
X₂₈ is selected from K, C, Q, or R,
X₃₀ is selected from I, F, or L,
X₃₁ is selected from R, M, or K,
X₃₂ is selected from D, N, K, or E, and
X₃₃ is selected from D, N, or E.

In some embodiments,
X₇ is R,
X₉ is F,
X₁₀ is Y,
X₁₃ is L,
X₂₃ is R, and/or
X₃₀ is I.

In some embodiments,
X₁₁ is E or D,
X₁₂ is A or T,
X₁₄ is H or W,
X₁₆ is P, A or L,
X₁₇ is N, L or T,
X₁₈ is N or M, and/or
X₂₃ is R.

In some embodiments,
X₁ is L,
X₄ is L, and/or
X₂₉ is Q, K, or R.

In some embodiments, the hyaluronidase has an amino acid sequence shown in any one of SEQ ID NOs. 6, 7, or 65 to 71, the sialidase has an amino acid sequence shown in any one of SEQ ID NOs. 4, 5, or 55 to 60, the collagenase has an amino acid sequence shown in SEQ ID NO. 8, the heparinase has an amino acid sequence shown in SEQ ID NO. 9, the chondrosulphatase has an amino acid sequence shown in SEQ ID NO. 10, and the chondroitinase has an amino acid sequence shown in SEQ ID NO. 11.

In some embodiments, the second moiety of the molecule is an antibody or an antigen-binding fragment thereof including heavy chain variable regions CDR1 to CDR3, and amino acid sequences of the heavy chain variable regions CDR1 to CDR3 respectively include SEQ ID NOs. 31 to 33 according to the Kabat numbering convention.

In an optional embodiment, the second moiety of the molecule includes a polypeptide having the amino acid sequence selected from any one of SEQ ID NOs. 12 to 18, and 34 to 39, or an Fc-binding fragment thereof.

In an optional embodiment, of the second moiety of the second molecule is selected from a polypeptide having the amino acid sequence as shown in any one of SEQ ID NOs. 34 to 39, or an Fc-binding fragment thereof.

In an optional embodiment, an amino acid sequence of the second moiety of the second molecule is SEQ ID NO. 34, 36, 38, or 39.

In some embodiments, the molecule is selected from a polypeptide represented by the amino acid sequence shown in any one of SEQ ID NOs. 19 to 30 or SEQ ID NOs. 61 to 64.

A third aspect of the present disclosure provides a polypeptide, in which the polypeptide is the second moiety of the second molecule in the targeting complex in any one of the embodiments described above, and the second moiety of the second molecule is the polypeptide having an amino acid mutation relative to the wild-type Z33.

In some embodiments, the polypeptide is a polypeptide having an amino acid mutation relative to the wild-type Z33. In some embodiments, a position of the amino acid mutation includes at least one of positions 1, 4 and 29, and the wild-type Z33 has an amino acid sequence shown in SEQ ID NO: 1. In some embodiments, the polypeptide having an amino acid mutation relative to the wild-type Z33 has an amino acid substitution relative to the wild-type Z33. In an optional embodiment, the substituted amino acid at the position 1 is selected from F or L. In an optional embodiment, the substituted amino acid at the position 4 is selected from Q, F, E, R, or L. In an optional embodiment, the substituted amino acid at the position 29 is selected from S, Q, R, or K.

In some embodiments, the amino acid sequence of the polypeptide having an amino acid mutation relative to the wild-type Z33 further has at least 50%, at least 60%, at least 70%, at least 80%, at least 85%, at least 90%, at least 92%, at least 95%, or at least 97% identity to the amino acid sequence shown in SEQ ID NO: 1.

In some embodiments, the polypeptide having an amino acid mutation relative to the wild-type Z33 includes the following amino acid sequence: X1X2X3X4X5X6X7X8X9X10X11X12X13X14X15X16X17X18X19X20X21X22X23X24X25X26X 27X28X29X30X31X32X33, where
X₁ is selected from F or L,
X₄ is selected from Q, F, E, R, or L,
X₂₉ is selected from S, Q, R, or K,
X₂ is selected from N, E, or S,
X₃ is selected from M, K, or E,
X₅ is selected from Q or C,
X₆ is selected from Q, W, L, K, or A,
X₇ is selected from R, L, K, N, A, D, or C,
X₈ is selected from R or A,
X₉ is selected from F, M, or A,
X₁₀ is selected from Y, A, or S,
X₁₁ is selected from E, A, D, or S,
X₁₂ is selected from A, I, T, or E,
X₁₃ is selected from L, A, G, or K,
X₁₄ is selected from H, A, V, F, W, or G,
X₁₅ is selected from D, V, C, N, or L,
X₁₆ is selected from P, L, or A,
X₁₇ is selected from N, L, T, or G,
X₁₈ is selected from L, M, or V,
X₁₉ is selected from N, G, or T,
X₂₀ is selected from E, K, A, or Q,
X₂₁ is selected from E or Q,
X₂₂ is Q,
X₂₃ is R,
X₂₄ is selected from N, W, A, or K,
X₂₅ is selected from A or G,
X₂₆ is selected from K, R, or F,
X₂₇ is I,
X₂₈ is selected from K, C, Q, or R,
X₃₀ is selected from I, F, or L,
X₃₁ is selected from R, M, or K,
X₃₂ is selected from D, N, K, or E, and
X₃₃ is selected from D, N, or E.

In some embodiments,
X₇ is R,
X₉ is F,
X₁₀ is Y,
X₁₃ is L,
X₂₃ is R, and/or
X₃₀ is I.

In some embodiments,
X₁₁ is E or D,
X₁₂ is A or T,
X₁₄ is H or W,
X₁₆ is P, A or L,
X₁₇ is N, L or T,
X₁₈ is N or M, and/or
X₂₃ is R.

In some embodiments,
X₁ is L,
X₄ is L, and/or
X₂₉ is Q, K, or R.

In an optional embodiment, the amino acid sequence of the polypeptide having an amino acid mutation relative to the wild-type Z33 includes an amino acid sequence selected from any one of SEQ ID NOs. 34 to 39. In an optional embodiment, the amino acid sequence of the polypeptide having an amino acid mutation relative to the wild-type Z33 is selected from any one of SEQ ID NOs. 34 to 39. In an optional embodiment, the amino acid sequence of the polypeptide having an amino acid mutation relative to the wild-type Z33 is SEQ ID NO. 34, 36, 38, or 39.

A fourth aspect of the present disclosure provides a fusion protein including the polypeptide having an amino acid mutation relative to a wild-type Z33. In an optional embodiment, the fusion protein includes the polypeptide having an amino acid mutation relative to the wild-type Z33 and an Asialoglycoprotein receptor (ASGPR)-binding peptide. In an optional embodiment, an amino acid sequence of the fusion protein includes an amino acid sequence selected from any one of SEQ ID NOs. 42 to 54. In an optional embodiment, the amino acid sequence of the fusion protein is selected from any one of SEQ ID NOs. 42 to 54.

A fifth aspect of the present disclosure provides a composition including: the targeting complex according to any one of the embodiments described above, the molecule according to any one of the embodiments described above, or the polypeptide according to any one of the embodiments described above; and a pharmaceutically acceptable carrier.

A sixth aspect of the present disclosure provides a nucleic acid encoding the targeting complex according to any one of the embodiments described above, the molecule according to any one of the embodiments described above, or the polypeptide according to any one of the embodiments described above.

A seventh aspect of the present disclosure provides an expression vector including the nucleic acid according to any one of the embodiments described above. In an optional embodiment, the expression vector includes the nucleic acid according to any one of the embodiments described above and a promoter operably linked to the nucleic acid.

An eighth aspect of the present disclosure provides a host cell including the nucleic acid as described above or the expression vector as described above.

In some embodiments, the host cell is a mammalian host cell, a prokaryotic host cell, or a yeast host cell.

A ninth aspect of the present disclosure provides a method including: administering, to an individual in need thereof, the targeting complex according to any one of the embodiments described above, the molecule according to any one of the embodiments described above, or the composition according to any one of the embodiments described above.

A tenth aspect of the present disclosure provides a method for treating cancer, the method including: administering, to an individual suffering from cancer, the targeting complex according to any one of the embodiments described above, the molecule according to any one of the embodiments described above, or the composition according to any one of the embodiments described above. In an optional embodiment, the cancer is breast cancer.

An eleventh aspect of the present disclosure provides a use of the targeting complex, the molecule, or the composition according to any one of the embodiments described above in the preparation of a medicament for treating a subject with cancer. In one embodiment, the use includes administering, to a subject suffering from cancer, the targeting complex according to any one of the embodiments described above, the molecule according to any one of the embodiments described above, or the composition according to any one of the embodiments described above. In an optional embodiment, the cancer is breast cancer.

In some embodiments, antitumor activity of the targeting complex is significantly superior to that of the first molecule in a targeting complex administered alone. In an optional embodiment, the targeting complex is selected from "Keytruda and NEU2-M-anFcVH", "Atezolizumab and Z33-mut1-PH20", "antibody-1 and NEU2-M-anFcVH", or "antibody-1 and PH20-anFcVH".

A twelfth aspect of the present disclosure provides a use of the polypeptide as described above, wherein the use is selected from:
(a) detecting an Fc-containing molecule, or
(b) purifying the Fc-containing molecule.

In an optional embodiment, the Fc-containing molecule is IgG.

Some of the embodiments provided by the present disclosure have the following advantages. The embodiments provide a universal technical platform that can make targeting molecules such as antibodies to direct extracellular molecule-modifying enzymes to the vicinity of target molecules, thereby effectively clearing enzyme substrate molecules around target cells or the target molecules. The synergy between the targeting molecules and enzymes enhances the therapeutic effect.

### DESCRIPTION OF THE DRAWINGS

FIG. 1 illustrates detection results of enzymatic activity of sialidase NEU2-M-anFcVH fusion protein.
FIG. 2 illustrates detection of enzymatic cleavage activity of a complex formed by NEU2-M-anFcVH and Keytruda on surfaces of Jurkat-T cells (PD-1 positive cells).
FIG. 3 illustrates detection of enzymatic cleavage activity of a complex formed by NEU2-M-anFcVH and antibody-1 on surfaces of SK-BR-3 cells (HER2 positive cells).
FIG. 4 illustrates detection results of enzymatic activity of a sialidase in a fusion protein formed by hNEU2-M and Z33.
FIG. 5 illustrates in vitro imaging results of the complex in tumors and organs of tumor-bearing mice.
FIG. 6 illustrates results of agarose gel electrophoresis after a substrate sodium hyaluronate is cleaved by a fusion protein.
FIG. 7 illustrates an efficacy result of each administration method of Keytruda + Neu2-M-anFcVH in CT26-hPDL1 model.
FIG. 8 illustrates an efficacy result of each administration method of Atezolizumab + PH20-anFcVH in PBMC NCI-H292 model.

### DETAILED DESCRIPTION OF THE INVENTION

### I. Definition

Unless otherwise defined, all technical and scientific terms used in the present disclosure have the same meaning as commonly understood by those skilled in the art. For the objects of the present disclosure, the following terms are defined.

The term "fusion protein" generally refers to a protein formed by the fusion of two or more proteins or polypeptides. The genes or nucleic acid molecules encoding the two or more proteins or polypeptides can be linked to each other to form a fusion gene or fused nucleic acid molecule, which can encode the fusion protein. The translation of the fusion gene produces a single polypeptide that possesses the properties of at least one, or even each, of the two or more proteins or polypeptides prior to fusion. The fusion protein described in the present disclosure generally includes at least two domains (A and B), and optionally includes a third component, such as a linker between the two domains. The production of recombinant fusion proteins is known in the art and generally involves removing a stop codon from a cDNA sequence encoding a first protein or polypeptide and then attaching to a cDNA sequence of a second protein by ligation or overlap extension PCR in an in-frame manner. The cDNA sequence is then expressed by cells as a single protein. The protein can be engineered to include the complete sequence of two original proteins or polypeptides, or only a portion of either.

The "targeting protein" refers to an antibody or fusion protein having a target-binding fragment moiety that is capable of binding to a target molecule. In some aspects, the targeting protein specifically binds to a cell surface molecule. As used in the present disclosure, a targeting protein that "specifically binds to a cell surface molecule" or "has specificity for a cell surface molecule" refers to a targeting protein that binds to a cell surface molecule with a greater affinity than other cell surface molecules. According to some embodiments, the targeting protein exhibits a binding affinity KD to a cell surface molecule of less than or equal to about 10⁻⁵ M, less than or equal to about 10⁻⁶ M, less than or equal to about 10⁻⁷ M, less than or equal to about 10⁻⁸ M, or less than or equal to about 10⁻⁹ M, 10⁻¹⁰ M, 10⁻¹¹ M, 10⁻¹² M, or less. Such affinity can be readily determined using techniques in the related art, such as by equilibrium dialysis, surface plasmon resonance (SPR) technology (for example, BIAcore2000 instrument, using general procedures outlined by manufacturers), radioimmunoassay, or by another method.

According to some embodiments, the targeting protein is an antibody. The terms "antibody" and "immunoglobulin" include antibodies or immunoglobulins of any isotype (for example, IgG (for example, IgG1, IgG2, IgG3, or IgG4), IgE, IgD, IgA, IgM, and the like), and whole antibodies (for example, an antibody composed of a tetramer, which is composed of two dimers of heavy and light chain polypeptides). The "antibody" and "immunoglobulin" include, but are not limited to, single-chain Fv, scFv, VHH, Fab, F(ab'), F(ab')₂, di-scFv, and affibody; chimeric antibodies; monoclonal antibodies, human antibodies, humanized antibodies (for example, humanized whole antibodies, humanized half antibodies, or humanized antibody fragments); and fusion proteins.

In some aspects, when the targeting protein is an antibody, the antibody can be a therapeutic antibody, for example, an antibody that has efficacy in the treatment of cancer (for example, through antibody-dependent cellular cytotoxicity and/or other mechanisms), immune-related disorders, and endothelial cell-related disorders, even in the absence of an extracellular molecule-modifying enzyme. For example, the antibody can be a therapeutic antibody that specifically binds to a tumor-associated cell surface molecule or a tumor-specific cell surface molecule.

In some aspects, when the targeting protein is an Fc-containing fusion protein, the portion thereof that binds to a target molecule is capable of specifically binding to the target molecule.

The "protein-protein interaction" refers to the non-covalent affinity or binding relation between proteins. The modes of protein-protein interaction include, but are not limited to, antigen-antibody binding, Protein A/Protein G-antibody Fc binding, and antibody Fc receptor-antibody Fc binding. The antigen-antibody binding includes the binding between antigens and antibodies, and the binding between antigen fragments (such as antigen epitopes) and antibodies and antigen-binding fragments thereof. The Protein A/Protein G-antibody Fc binding includes the binding between Protein A/Protein G and antibody Fc, and the binding between an Fc-binding fragment of Protein A/Protein G and antibody Fc. The antibody Fc receptor-antibody Fc binding includes the binding between antibody Fc receptor and antibody Fc, and the binding between an Fc-binding fragment of antibody Fc receptor and antibody Fc.

The "enzyme" refers to a class of proteins with catalytic ability.

The "extracellular molecule-modifying enzyme" is included in the targeting complex of the present disclosure as described above. As used in the present disclosure, the "extracellular molecule-modifying enzyme" is an enzyme that, upon binding of a targeting protein to a corresponding cell surface molecule of a target cell, causes a structural modification or change in one or more substrate molecules of the modifying enzyme on a surface of the target cell or in an extracellular matrix of the target cell. In some aspects, the structural change is a change in the structure of a cell surface molecule bound by the targeting protein. In other aspects, the structural change is a change in the structure of a molecule on the surface of the target cell other than the cell surface molecule bound by the targeting protein or an extracellular matrix molecule.

In some aspects, the extracellular molecule-modifying enzyme is a wild-type enzyme (that is, a naturally occurring enzyme). In other aspects, the enzyme is not a wild-type enzyme. For example, the extracellular molecule-modifying enzyme may be a non-native derivative of a wild-type enzyme. Such a derivative at least partially retains the enzymatic activity of the corresponding wild-type enzyme. Enzyme derivatives that can be used include those having fewer or more amino acids than the corresponding wild-type enzyme. Alternatively or additionally, an enzyme derivative may include one or more amino acid substitutions or amino acid modifications relative to the corresponding wild-type enzyme.

An example of a structural change effected by an extracellular molecule-modifying enzyme in a molecule on a surface of a target cell is cleavage of the molecule. In some aspects, the molecule cleaved by the extracellular molecule-modifying enzyme is a polymer. Cell surface polymers that can be cleaved (for example, degraded) by extracellular molecule-modifying enzymes include, but are not limited to, polypeptides, polysaccharides, glycoproteins, and the like. For example, the extracellular molecule-modifying enzyme can be a protease that cleaves a polypeptide (or a subset of interest thereof) on a surface of a target cell. In some aspects, a polymer cleaved by the extracellular molecule-modifying enzyme is a polysaccharide (or "glycan"), that is, a molecule containing glycosidically linked monosaccharides. In such an embodiment, the extracellular molecule-modifying enzyme may be, for example, a glycoside hydrolase (for example, hyaluronidase).

According to some embodiments, an extracellular molecule-modifying enzyme cleaves (for example, hydrolyzes) a terminal residue of a molecule (for example, a polymer) on a surface of a target cell. In some aspects, the terminal residue is present in molecules selected from the group consisting of oligosaccharides, polysaccharides, glycoproteins, glycolipids, and gangliosides. In some embodiments, the terminal residue is a terminal sialic acid residue. In this case, the extracellular molecule-modifying enzyme may be a sialidase (or a derivative thereof as described above) that cleaves the glycosidic bond of sialic acid (neuraminic acid) to release the terminal sialic acid residue from oligosaccharides, polysaccharides, glycoproteins, glycolipids, and other substrates.

According to some embodiments, an extracellular molecule-modifying enzyme cleaves (for example, hydrolyzes) a molecule (for example, a polymer) outside of a target cell. In some aspects, an extracellular molecule is a molecule present in an extracellular matrix (ECM) outside of a target cell, such as hyaluronic acid, collagen, heparin, chondroitin sulfate, chondroitin, and the like.

Hyaluronic acid (HA) is primarily found in mammalian connective tissue, skin, cartilage, and synovial fluid. Hyaluronic acid is also a major component of the vitreous humor of the eye. In connective tissue, the hydration associated with hyaluronic acid creates spaces between tissues, creating a favorable environment for cell movement and proliferation. Hyaluronic acid plays a key role in biological phenomena related to cell movement, including rapid development, regeneration, repair, embryogenesis, embryonic development, wound healing, angiogenesis and tumorigenesis (Toole 1991 Cell Biol. Extracell. Matrix, Hay (ed.), Plenum Press, New York, 1384-1386; Bertrand et al., 1992 Int. J. Cancer 52:1-6; Knudson et al., 1993 FASEB J. 7:1233-1241). In addition, hyaluronic acid levels are associated with tumor invasion (Ozello et al., 1960 Cancer Res. 20:600-604; Takeuchi et al., 1976, Cancer Res. 36:2133-2139; Kimata et al., 1983 Cancer Res. 43:1347-1354).

The "hyaluronidase" is a type of glycosidase capable of degrading hyaluronic acid and some glycosaminoglycans. The hyaluronidase can be found throughout the animal kingdom and includes wild-type hyaluronidases and mutant hyaluronidases.

There are three major classes of hyaluronidases.
1. Mammalian hyaluronidases (EC 3.2.1.35) are endo-β-N-acetylhexosaminidases that primarily produce tetrasaccharides and hexoses as end products. They have hydrolytic and transglycosidase activities and can degrade hyaluronic acid and chondroitin sulfate (CS), especially C4-S and C6-S.
2. Bacterial hyaluronidases (EC 4.2.99.1) degrade hyaluronic acid and, to varying degrees, CS and DS. They are endo-β-N-acetylglucosaminidases that act via β-elimination reactions, primarily producing disaccharide end products.
3. Hyaluronidases (EC 3.2.1.36) from leeches, other parasites, and crustaceans are endo-β-glucuronidases that hydrolyze β1-3 linkages to produce tetrasaccharide and hexose end products.

Mammalian hyaluronidases can be further divided into two groups: neutrally active enzymes and acid-active enzymes. There are six hyaluronidase-like genes in the human genome: HYAL1, HYAL2, HYAL3, HYAL4, HYALP1 and PH20/SPAM1. HYALP1 is a pseudogene and HYAL3 shows no enzymatic activity against any known substrates. HYAL4 is chondroitinase that lacks activity towards hyaluronic acid. HYAL1 is a prototype acid-active enzyme and PH20 is a prototype neutral-active enzyme. Acidic-active hyaluronidases, such as HYAL1 and HYAL2, lack catalytic activity in neutral pH. For example, HYAL1 is catalytically inactive in vitro above pH 4.5 (Frost et al., Anal Biochemistry, 1997). HYAL2 is an acid-active enzyme with very low specific activity in vitro.

Hyaluronidase-like enzymes can also be characterized as those that are locked to a plasma membrane via a glycosylphosphatidylinositol anchor. However, there are variations between species. For example, bovine PH20 is very loosely attached to a plasma membrane and is not anchored by a phospholipase sensitive anchor (Lalancette et al., Biol Reprod. 2001 Aug; 65(2):628-36). This unique feature of bovine hyaluronidase makes it possible to use soluble bovine testicular hyaluronidase as an extract for clinical applications. Other PH20s are lipid-anchored enzymes that are insoluble without the application of detergents or lipases. For example, human PH20 is anchored to a plasma membrane via a GPI anchor. Attempts have been made to prepare DNA constructs of human PH20 that do not incorporate a lipid anchor, but have resulted in catalytically inactive or insoluble enzymes (Arming et al., Eur J Biochem. 1997 Aug 1; 247(3):810-4). Naturally occurring cynomolgus monkey sperm hyaluronidase was found to be both soluble and membrane-bound. The 64 kDa membrane-bound form is enzymatically active at pH 7.0, whereas the 54 kDa form is active only at pH 4.0 (Cherr et al., Dev Biol. 1996 Apr 10; 175(1):142-53). Thus, the soluble form of PH20 lacks enzymatic activity under neutral conditions. Examples of hyaluronidases include, but are not limited to, those disclosed in WO2020022791A1 and WO2013102144A2.

Chondroitinases are enzymes found throughout the animal kingdom. These enzymes degrade glycosaminoglycans via endoglycosidase reactions. Specific examples of known chondroitinases include chondroitinase ABC (from *Proteus vulgaris;* Japanese Patent Laid-Open No. 6-153947, T. Yamagata, H. Saito, O. Habuchi and S. Suzuki, J. Biol. Chem., 243, 1523 (1968), S. Suzuki, H. Saito, T. Yamagata, K. Anno, N. Seno, Y. Kawai and T. Furuhashi, J.Biol. Chem., 243, 1543 (1968)); chondroitinase AC (from *Flavobacterium heparinum;* T. Yamagata, H. Saito, O. Habuchi and S. Suzuki, J. Biol. Chem., 243, 1523(1968)); chondroitinase ACII (from *Arthrobacter aurescens;* K. Hiyama and S. Okada, J. Biol. Chem., 250, 1824 (1975), K. Hiyama and S. Okada, J. Biochem. (Tokyo), 80, 1201 (1976)); hyaluronidase ACIII (from *Flavobacterium sp.* Hp102; Hirofumi Miyazono, Hiroshi Kikuchi, Keiichi Yoshida, Kiyoshi Morikawa and Kiyochika Tokuyasu, Seikagaku, 61,1023 (1989)); chondroitinase B (from *Flavobacterium heparinum;* YM. Michelacci and C.P. Dietrich, Biochem. Biophys. Res. Commun., 56, 973 (1974), Y.M. Michelacci and C.P. Dietrich, Biochem. J., 151, 121 (1975), Kenichi Maeyama, Akira Tawada, Akiko Ueno and Keiichi Yoshida, Seikagaku, 57, 1189 (1985)); chondroitinase C (from *Flavobacterium sp.* Hp102; Hirofumi Miyazono, Hiroshi Kikuchi, Kelichi Yoshida, Kiyoshi Morikawa and Kiyochika Tokuyasu, Seikagaku, 61,1023 (1939)) and analogs thereof.

The "chondrosulphatase" (hereinafter, sometimes referred to as "ChSase") is a type of enzyme that degrades glycosaminoglycans such as chondroitin sulfate, chondroitin, and hyaluronic acid into unsaturated disaccharides (ΔDi and oligosaccharides).

The "heparinase" is a polysaccharide-cleaving enzyme that acts on heparin or heparan sulfate. It exists in many microorganisms, among which the heparinase from Flavobacterium heparinum is the most common. There are only three types of heparinase from Flavobacterium heparinum: heparinase I (EC 4.2.2.7), heparinase II (No EC code), and heparinase III (EC 4.2.2.8) (Robert J. Linhardt et al., Purification and characterization of heparin lyases from Flavobacterium heparinum, JBC 1992 Vol. 267:24347-24355).

Heparinase III mainly acts on heparan sulfate and has a molecular weight of 73 kDa. Compared with the other two heparinases, studies have shown that heparinase III can act on heparinoids in the extracellular matrix to produce active heparin small molecules, which can inhibit the proliferation of capillary epithelial cells, thereby inhibiting the growth of tumor cells and reducing the metastasis and spread of cancer cells (see US6869789B2 and CN114703168A).

The collagenase is an endopeptidase that specifically recognizes the Pro-X-Gly-Pro sequence (which occurs frequently in collagen and is rarely found in other proteins) and cleaves the peptide bond between the neutral amino acid (X) and glycine (Gly) in this sequence. Many proteases can hydrolyze single-chain, denatured collagen polypeptides, but collagenase is the only protease that can degrade native collagen fibrils with a triple-stranded superhelical structure, which are widely present in connective tissue. Common collagenases include collagenase 1 (MMP1), collagenase 2 (MMP8) and/or collagenase 3 (MMP13), serine protease Cathepsin L, and the like.

The "sialidase" (EC 3.2.1.18, neuraminidase) catalyzes the hydrolysis of terminal sialic acid residues in glycoconjugates. The sialidases and polypeptides having sialidase activity described in patent applications WO2018006034A1, WO2019136167A1, WO2022150521A, WO2022150516A1, WO2022150507A1, WO2022006492A2, WO2021003469A2, and WO2021003468A are all incorporated into the present disclosure.

The "targeting complex" refers to a protein complex formed by non-covalently linking a targeting protein containing an antibody Fc fragment moiety with a fusion protein of an extracellular molecule-modifying enzyme and a targeting protein-binding fragment. The formation of this complex does not affect or lose the binding effect between the targeting protein and its target molecule, and does not affect or lose the degradation activity of the extracellular molecule-modifying enzyme on an extracellular molecule.

The "target cells" refer to specific cell populations that are actively identified and targeted in drug, gene, or cell therapy. They are specifically bound by corresponding targeting proteins, ligands or modifying enzymes through surface markers, metabolic characteristics or functional states, thereby achieving precise intervention. Depending on the type of the target cell, the corresponding targeting protein and extracellular molecule-modifying enzyme can be selected. According to some examples, the target cells are selected from cancer cells, immune cells, endothelial cells, and epithelial cells. Target cells of interest include, but are not limited to, cells associated with a particular disease or condition. For example, the target cells may be normally functioning cells (for example, normally functioning immune cells), and the extracellular molecule-modifying enzyme modulates the function of the cells in a manner that is therapeutically effective for an individual in need thereof, for example, enhancing the function of the cells, which is beneficial in treating the disease of the individual.

In other aspects, the target cells are not normal cells. Abnormal target cells of interest include, but are not limited to, cancer cells. The "cancer cells" refer to cells that exhibit a tumor cell phenotype, which can be characterized by, for example, abnormal cell growth, abnormal cell proliferation, loss of density-dependent growth inhibition, anchorage-independent growth potential, the ability to promote tumor growth and/or development in immunocompromised non-human animal models, and/or any appropriate indicator of cell transformation. The "cancer cells" are used interchangeably in the present disclosure with "tumor cells", "malignant cells", or "cancerous cells", and include cancer cells of solid tumors, semi-solid tumors, primary tumors, metastatic tumors, and the like. In some aspects, the cancer cells are carcinoma cells. According to some embodiments, the cancer cells are selected from breast cancer cells, ovarian cancer cells, gastric cancer cells, colon cancer cells, and the like.

In some aspects, when a target cell is a cancer cell, a molecule on a surface of the cancer cell to which a targeting protein binds is a tumor-associated cell surface molecule or a tumor-specific cell surface molecule. The "tumor-associated cell surface molecule" refers to a cell surface molecule that has limited expression on cells of normal tissues and is expressed on malignant cells, or a cell surface molecule that is expressed at a higher density on malignant cells than on normal cells, such as, but not limited to, PD-L1 or HER2, or a molecule that is specifically expressed during embryonic development, is silenced in adults, but is reactivated in tumors.

In some aspects, when a target cell is an immune cell, a molecule on a surface of the immune cell to which a targeting protein binds is a cell surface molecule associated with tumor immune recognition or a surface molecule associated with tumor immune escape, such as but not limited to PD-1.

The "second moiety non-covalently binding to a constant region of a protein molecule" refers to a protein, an antibody, an Fc-binding fragment of an antibody, or an Fc-binding fragment of a polypeptide that can bind to a constant region of an antibody (including a CH region (including an Fc region, a CH1 region, a hinge region) and/or CL region of an antibody), an Fc fragment moiety, or a protein containing an antibody Fc fragment moiety in a non-covalent manner, and includes, but not limited to, Protein A, Protein G, Fc receptor (FcR), an antibody that can specifically bind to a constant region fragment of an antibody, other Fc-binding peptides, and Fc-binding fragments of the above proteins or polypeptides. The Fc-binding proteins or fragments thereof disclosed in the article published by Weonu Choe et al. in 2016 (Weonu Choe et al., Fc-Binding Ligands of Immunoglobulin G: An Overview of High Affinity Proteins and Peptides. Materials (Basel). 2016 Dec; 9(12): 994.) are all introduced into the present disclosure.

"Protein A", also known as staphylococcal protein A, refers to a cell wall-anchored surface protein from *Staphylococcus aureus* that enables the bacterium to evade innate and adaptive immune responses. Protein A binds to the Fc portion of immunoglobulins. Protein A and Fc-binding fragments thereof disclosed in the related art, such as CN106422418A and EP2655404A1, are incorporated into the present disclosure. An exemplary Fc-binding fragment of Protein A, such as Z33, has an amino acid sequence shown in SEQ ID NO. 18. This is an Fc-binding fragment of Protein A that can bind to Fc when present alone. Fc-binding peptides such as those shown in SEQ ID NOs. 16 and 17 are other polypeptides capable of binding to Fc.

"Protein G", also known as Streptococcal Protein G (SPG), is a protein isolated from the cell wall and culture supernatant of *streptococci* that can bind to antibody Fc. Protein G and Fc-binding fragments thereof disclosed in the related art, such as US5108894A and EP1054061A1, are incorporated into the present disclosure.

FcR is a type of cell surface protein that specifically binds to an Fc fragment moiety of an antibody. FcRs that bind to IgG antibodies include Fc-gamma receptor (FcγR) and Fc receptor neonatal (FcRn).

The term "about," when used in conjunction with a numerical value, is intended to encompass numerical values within a range having a lower limit that is 5% less than the specified numerical value and an upper limit that is 5% greater than the specified numerical value.

The term "and/or" should be understood to mean any one of the options or a combination of any two or more of the options.

As used in the present disclosure, the term "comprising", "comprises", "comprise", "comprised", "including", "includes", "include", and/or "included" are meant to include the recited elements, integers or steps, but not to exclude any other elements, integers or steps. In the present disclosure, when the term "comprising", "comprises", "comprise", "comprised", "including", "includes", "include", and/or "included" is used, unless otherwise specified, it also encompasses the case where it consists of the recited elements, integers or steps. For example, when referring to a variable region of an antibody "comprising", "comprises", "comprise", "comprised", "including", "includes", "include", and/or "included" a particular sequence, it is intended to also encompass a variable region of an antibody consisting of the particular sequence.

In the present disclosure, the term "antibody" refers to a polypeptide comprising at least a variable region of a light chain or a heavy chain immunoglobulin that specifically recognizes and binds to an antigen. This term encompasses various antibody structures, including but not limited to monoclonal antibodies, polyclonal antibodies, single-chain antibodies or multi-chain antibodies, monospecific or multispecific antibodies (for example, bispecific antibodies), fully human antibodies or chimeric antibodies or humanized antibodies, full-length antibodies and antibody fragments, antibody conjugates, so long as they exhibit the desired antigen-binding activity.

The term "antigen-binding fragment" of an antibody (which is used interchangeably with "antibody fragment" and "antigen-binding portion" in the present disclosure) refers to a molecule that is not an intact antibody, but includes a portion of an intact antibody that binds to an antigen to which the intact antibody binds. As understood by those skilled in the art, the antigen-binding portion of an antibody typically includes amino acid residues from a "complementarity determining region" or "CDR". Antigen binding fragments can be prepared using recombinant DNA technology or by enzyme or chemical cleavage of intact antibodies. Antigen-binding fragments include, but are not limited to, Fab, scFv, F(ab'), F(ab')₂, Fab'-SH, Fv, single-chain Fv, diabody, triabody, tetrabody, minibody, and single-domain antibody VHH. For a more detailed description of antibody fragments, see Fundamental Immunology, edited by W. E. Paul, Raven Press, N.Y. (1993); Shao Rongguang et al. (eds.), Antibody Drug Research and Application, The People's Health Press Co., Ltd (2013); Hollinger et al., PNAS USA 90:6444-6448 (1993); Hudson et al., Nat. Med. 9:129-134 (2003).

The term "variable region" or "variable domain" refers to the domain of a heavy or light chain of an antibody that is involved in binding the antibody to an antigen. The variable domains of the heavy and light chains of native antibodies generally have similar structures, with each domain including four conserved framework regions (FRs) and three complementarity determining regions (see, for example, Kindt et al. Kuby Immunology, 6th ed., W. H. Freeman and Co. p. 91 (2007)). A single VH or VL domain may be sufficient to confer antigen-binding specificity. In addition, an antibody that binds to a particular antigen can be isolated using a VH or VL domain from the antibody to screen libraries of complementary VL or VH domains, respectively. See, for example, Portolano et al., J. Immunol. 150:880-887 (1993); Clarkson et al., Nature 352:624-628 (1991).

The variable region typically exhibits the same general structure of a relatively conserved framework region (FR) connected by three hypervariable regions, which are also called complementarity determining regions or CDRs. The CDRs from the two chains of each pair are usually aligned by the framework regions, which allow binding to specific epitopes. The two light chain and heavy chain variable regions typically include the domains FR1, CDR1, FR2, CDR2, FR3, CDR3, and FR4 from N-terminus to C-terminus. The portion of an antibody outside the variable region is the constant region of the antibody. In a fusion protein that includes an Fc fragment moiety and has target-binding ability, the constant region of the antibody is the Fc fragment moiety included in the fusion protein.

The "pharmaceutical composition" refers to a formulation or combination of formulations containing one, two, or more active ingredients, in which the active ingredients contained herein are present in a biologically effective form and do not contain any other ingredients that would cause unacceptable toxicity to a subject who are given the formulation. When a "pharmaceutical composition" exists in combination as a combination of individual formulations containing two or more different active ingredients, it can be administered simultaneously, sequentially, separately, or at intervals, with the aim of exerting the biological activity of multiple active ingredients together for the treatment of diseases.

In a specific aspect, the bifunctional molecule according to the present disclosure further includes a peptide linker that connects the antigen-binding domain and IL-7m to an Fc chain. The peptide linker typically has sufficient length and flexibility to ensure that IL-7m and the linker-connected antigen-binding domain have sufficient spatial freedom to perform their functions.

The "linker", "peptide linker", or "connector" refers to a sequence of at least one amino acid. Such peptide linkers can be used to prevent steric hindrance. Peptide linkers are typically 3 to 44 amino acid residues long. Preferably, the peptide linker has 3 to 30 amino acid residues. In some aspects, the peptide linker has 3, 4, 5, 6, 7, 8, 9, 10, 11, 12, 13, 14, 15, 16, 17, 18, 19, 20, 21, 22, 23, 24, 25, 26, 27, 28, 29, or 30 amino acid residues.

The peptide linker sequence may be a naturally occurring sequence or a non-naturally occurring sequence. If used for therapeutic purposes, the peptide linker is preferably non-immunogenic in subjects who are given the bifunctional molecule. One set of useful peptide linker sequences is those derived from the hinge region of heavy chain antibodies, as described in WO96/34103 and WO94/04678. Another set of useful peptide linker sequences is those derived from "peptide linkers" as described in CN110520444A. Other examples are polyalanine linker sequences.

Further preferred examples of linker sequences are flexible linkers (for example, (Gly4Ser)ₙ, (Gly)ₙ), rigid linkers (for example, (EAAAK)n, (XP)n), and cleavable linkers. For example, Gly/Ser linkers of different lengths include (Gly4Ser)ₙ (n = 1 to 9), (Gly4Ser)₄, (Gly4Ser)₃, (Gly4Ser)₂, Gly4Ser, Gly3Ser, Gly3, Gly2ser, and (Gly3Ser2)₃, particularly (Gly4Ser)₃. Preferably, the peptide linker is selected from (Gly4Ser)₄, (Gly4Ser)₅, (Gly4Ser)₃, and (Gly3Ser2)₃. More preferably, the peptide linker is (Gly4Ser)₅.

In one aspect, the peptide linker included in a fusion protein is selected from (Gly4Ser)₄, (Gly4Ser)₃, (Gly4Ser)₂, Gly4Ser, Gly3Ser, Gly3, Gly2ser and (Gly3Ser2)₃, preferably (Gly4Ser)₃. Preferably, the peptide linker is selected from (Gly4Ser)₅, (Gly4Ser)₄, (Gly4Ser)₃, and (Gly3Ser2)₃.

The "effective dose" is generally an amount sufficient to reduce the severity and/or frequency of symptoms, eliminate such symptoms and/or underlying causes, prevent the onset of symptoms and/or their underlying causes, and/or ameliorate or improve the impairment caused by or associated with a disease state (for example, lung disease). In some examples, the effective dose is a therapeutically effective dose or a preventatively effective dose. The "therapeutically effective dose" is an amount sufficient to treat a disease state or symptom, especially a state or symptom associated with the disease state, or otherwise prevent, hinder, delay or reverse the progression of the disease state or any other undesirable symptom associated with the disease in any way. The "preventatively effective dose" is an amount that, when administered to a subject, has a predetermined preventative effect, such as preventing or delaying the onset (or recurrence) of the disease state, or reducing the likelihood of the onset (or recurrence) of the disease state or related symptoms. A complete therapeutic or preventive effect may not occur with a single dose, but may only occur after a series of doses. Therefore, the therapeutically effective dose or preventatively effective dose can be administered once or multiple times.

The terms "host cell", "host cell line", and "host cell culture" are used interchangeably and refer to cells in which exogenous nucleic acids are introduced, including the progeny of such cells. Host cells include "transformants" and "transformed cells," which include primary transformed cells and progeny thereof, regardless of the number of passages. The progeny may not be identical to parental cells in terms of nucleic acid content and may contain mutations. The present disclosure includes mutant progeny that have the same function or biological activity as those screened or selected in the initially transformed cells.

The "transfection" refers to the introduction of exogenous nucleic acids into eukaryotic cells. The transfection can be achieved through various methods known in the art, including electroporation, microinjection, and liposome fusion.

"Nucleic acid molecule encoding", "encoding DNA sequence", and "encoding DNA" refer to the order of deoxyribonucleotides along a deoxyribonucleic acid strand. This sequence of deoxyribonucleotides determines the sequence of amino acids along a polypeptide (protein) chain. Therefore, the nucleic acid sequence encodes the amino acid sequence.

As used in the present disclosure, the term "tumor" refers to a disease characterized by the pathological proliferation of cells or tissues, and their subsequent migration or invasion of other tissues or organs. Tumor growth is typically uncontrolled and progressive, without inducing or inhibiting normal cell proliferation. Tumor includes "cancer" and refers broadly to all malignant tumors.

"Treatment" refers to clinical intervention in an attempt to alter the disease process in an individual or in the cells causing the disease, either for prevention or to intervene in the clinical pathological process. The therapeutic effects include, but are not limited to, preventing the occurrence or recurrence of the disease, alleviating symptoms, reducing any direct or indirect pathological consequences of the disease, preventing metastasis, slowing the progression of the disease, improving or alleviating the condition, relieving or improving the prognosis, and the like.

"Combination" refers to a treatment regimen that provides at least two or more different therapies to achieve a specified therapeutic effect. These therapies can be physical, such as radiation therapy, or chemical, such as administering a drug to a subject, including combination drugs. "Combination drugs" refer to a combination of two or more pharmaceutical preparations, each containing an active ingredient, that are used in combination when administered to a subject. The active ingredients can be mixed together to form a single dosing unit or administered separately as independent dosing units. During administration, different pharmaceutical preparations can be given substantially simultaneously, concurrently, or sequentially.

The term "pharmaceutically acceptable" refers to compounds, materials, compositions and/or dosage forms that are suitable for use in contact with the tissues of humans and animals without excessive toxicity, irritation, allergic response, or other problems or complications, commensurate with a reasonable benefit/risk ratio.

As used in the present disclosure, "anti-PD1 antibody" refers to an antibody or an antigen-binding fragment thereof that targets the immune checkpoint protein PD1 (or PD-1) and can block or affect the binding of PD1 to a ligand thereof (such as PD-L1). Optional anti-PD1 antibodies include, but are not limited to, Nivolumab and keytruda^{®} (Pembrolizum-ab). "PD1 (programmed death receptor 1)", also known as CD279, belongs to the immunoglobulin superfamily.

The term "amino acid mutation" encompasses amino acid substitution, deletion, insertion, and modification. Any combination of substitutions, deletions, insertions, and modifications can be used to achieve the final construct, as long as the final construct has the desired properties. Amino acid sequence deletions and insertions include amino and/or carboxyl terminus deletions and amino acid insertions. In some embodiments of the present disclosure, a specific amino acid mutation is an amino acid substitution. In one embodiment, the amino acid mutation is a non-conservative amino acid substitution, that is, replacing one amino acid with another amino acid that has a different structure and/or chemical properties. The amino acid substitution includes replacement with non-naturally occurring amino acids or with derivatives of 20 naturally occurring amino acids (for example, 4-hydroxyproline, 3-methylhistidine, ornithine, homoserine, 5-hydroxylysine). Amino acid mutations can be generated using genetic or chemical methods known in the art. Genetic methods may include site-directed mutagenesis, PCR, gene synthesis, and the like. Methods of altering amino acid side chain groups other than genetic engineering, such as chemical modification, are also expected to be available. Various names may be used in the present disclosure to refer to the same amino acid mutation.

The term "affinity" or "binding affinity" refers to the strength of the specific binding between two biomolecules (such as ligand and receptor, antibody and antigen, enzyme and substrate) through non-covalent interactions (such as hydrogen bonds, van der Waals forces, hydrophobic interactions, electrostatic attraction, and the like), and is usually quantitatively characterized by the equilibrium dissociation constant (KD). The smaller the KD value (for example, from picomolar to nanomolar), the tighter the intermolecular binding and the higher the affinity. For example, in antibody engineering, the affinity of the Fc region to the protein A/Z33 mutant directly affects antibody purification efficiency, while in the development of therapeutic antibodies, high affinity can enhance target binding ability and prolong the residence time of drugs in target tissues, thereby improving efficacy. The affinity can be accurately measured using techniques such as surface plasmon resonance (SPR), isothermal titration calorimetry (ITC), and bio-layer interferometry (BLI), with bio-layer interferometry (BLI) being the preferred method.

The term "polynucleotide" is used interchangeably with the term "nucleic acid" in the present disclosure and refers to deoxyribonucleotides or ribonucleotides in single-stranded or double-stranded form, and polymers thereof. The term encompasses nucleic acids containing known nucleotide analogs or modified backbone residues or linkages, which are synthetic, naturally occurring, or non-naturally occurring, have binding properties similar to a reference nucleic acid, and are metabolized in a manner similar to that of a reference nucleotide. Examples of such analogs include, but are not limited to, organophosphorothioates, phosphoramidates, methylphosphonates, chiral methylphosphonates, 2'-O-methyl ribonucleotides, and peptide-nucleic acids (PNAs).

The term sequence "identity" refers to the degree (percentage) to which amino acids/nucleic acids of two sequences are identical at equivalent positions when two sequences are optimally aligned (gaps are introduced where necessary to obtain the maximum percentage of sequence identity, and no conserved substitutions are considered part of sequence identity). Those skilled in the art can determine the parameters suitable for measuring alignment, including any algorithms required to achieve maximum alignment across the full length of the sequences being compared. For example, the PILEUP and BLAST algorithms can be used to compute identity or alignment sequences (such as identifying equivalent or corresponding sequences, typically at their default settings). The software used for BLAST analysis is publicly available from China National Center for Bioinformation (http//www.ncbi.nIm.nih.gov/). This algorithm first identifies high-scoring sequence pairs (HSPs) by recognizing short characters of length W in the query sequence that match or satisfy some positive threshold score T when compared with characters of the same length in the database sequence. T is called the neighborhood word score threshold. These initial neighborhood wordhits serve as seeds to initiate the search for HSPs containing them. Wordhits expand in both directions along each sequence as long as the cumulative alignment score can be increased. The expansion of a wordhit in each direction stops when the cumulative alignment score decreases by an amount X from its maximum realized value, when the cumulative score reaches zero or below due to the accumulation of one or more negative score residue alignments, or when the end of either sequence is reached. The BLAST algorithm parameters W, T, and X determine the sensitivity and speed of the alignment. The BLAST program uses a word length (W) of 11, a BLOSUM62 scoring matrix alignment (B) of 50, an expectation (E) of 10, M = 5, N = 4, and a comparison of both strands as defaults.

The BLAST algorithm performs statistical analysis on the similarity between two sequences. One similarity metric provided by the BLAST algorithm is the minimum sum probability (P(N)), which indicates the probability that a match between two polynucleotide or amino acid sequences will occur by chance. For example, a sequence is considered similar to another sequence if the minimum sum probability of comparing a first sequence with a second sequence is less than about 1, preferably less than about 0.1, more preferably less than about 0.01, and most preferably less than about 0.001. Alternatively, the UWGCG software package provides the BESTFIT procedure (for example, on its default settings) for calculating identity.

Based on common knowledge in the field, the above preferred conditions can be freely combined to obtain various preferred embodiments of the present disclosure. All reagents and raw materials used in the present disclosure are commercially available.
Examples The technical solutions of the present disclosure are further described below through specific embodiments. Those skilled in the art should understand that the embodiments described are merely to help understand the present disclosure and should not be regarded as specific limitations on the present disclosure.

For experimental methods in the following examples where specific conditions are not specified, follow methods and conditions in the related art or the product instructions.

### Example 1: Construction, Expression and Preparation of Fusion Proteins

DNA fragments encoding fusion proteins were synthesized from the whole genome (amino acid sequences of different proteins are shown in Table 1) and cloned into the constructed expression vectors. The expression vectors consisted of the following components.
(1) Glutamine synthase gene, used as a selection marker;
(2) Origin of replication: ori, which allows this plasmid to replicate in E. coli;
(3) β-lactamase gene, which confers ampicillin resistance in E. coli;
(4) Enhancer and promoter from human cytomegalovirus;
(5) Human 1-immunoglobulin poly-adenylation ("poly A") signal sequence.

As described above, the gene of interest containing the fusion proteins was prepared by gene synthesis and cloned into pDrG(Amp) plasmid using Hind III and Pac I.

The constructed fusion protein vectors were transformed into *E. coli* DH5α, and positive clones were selected and inoculated into 500 ml of LB medium for amplification. DNA was extracted and purified using the Ultrapure Plasmid DNA Purification Kit from Qiagen according to the manufacturer's instructions. The plasmid DNA containing the fusion protein coding sequences was transfected into CHO-K1 (Chinese hamster ovary cells, purchased from ATCC) using an X-porator H1 electroporator from Yida Biotechnology Co., Ltd. The operation method was carried out according to the manufacturer's instructions.

24 to 48 hours after transfection, the cell culture medium was replaced with a selection medium containing a selection drug. This selection medium was changed every 3 to 4 days until cell viability exceeded 95%. The volume of cells was then expanded for expression culture, and the cells were transferred to an expression medium for batch culture. After 3 to 4 days of culture, a feed medium and glucose were added every other day. When the cell density exceeded 1 × 10⁷ cells/mL, the temperature was lowered to 31°C for further culture. When the cell viability fell below 80%, the culture supernatant was collected by centrifugation at 9500 rpm for 20 minutes, and then purified using nickel affinity chromatography to obtain fusion protein samples. The purified samples were sterilized by filtration through a 0.22 µm membrane, and the protein concentration was detected using nanodrop. The samples were stored at 2°C to 8°C.

**Table 1 Amino acid sequences of enzymes, antibodies, and Fc-binding polypeptides**

| **Name** | **Sequence** |
|---|---|
| Antibody-1 light chain (SEQ ID NO. 1) | DIQMTQSPSSLSASVGDRVTITCRASQDVSTAVAWYQQKPGKAPKLLI YSASYLYSGVPSRFSGSRSGTDFTLTISSLQPEDFATYYCQQHYTTPPT FGQGTKVEIKRTVAAPSVFIFPPSDEQLKSGTASVVCLLNNFYPREAK VQWKVDNALQSGNSQESVTEQDSKDSTYSLSSTLTLSKADYEKHKV YACEVTHQGLSSPVTKSFNRGEC |
| Antibody-1 first heavy chain (SEQ ID NO. 2) | EVQLVESGGGLVQPGGSLRLSCAASGFNIKDTYIHWVRQAPGKGLE WVARIYPTNGYTRYADSVKGRFTISADTSKNTAYLQMNSLRAEDTAV YYCSRWGGEGFYAMDYWGQGTLVTVSSASTKGPSVFPLAPSSKSTS GGTAALGCLVKDYFPEPVTVSWNSGALTSGVHTFPAVLQSSGLYSLSS VVTVPSSSLGTQTYICNVNHKPSNTKVDKKVEPKSCDKTHTCPPCPA PELLGGPSVFLFPPKPKDTLMISRTPEVTCVVVDVSHEDPEVKFNWY VDGVEVHNAKTKPREEQYNSTYRVVSVLTVLHQDWLNGKEYKCKV SNKALPAPIEKTISKAKGQPREPQVYTLPPCREEMTKNQVSLWCLVK GFYPSDIAVEWESNGQPENNYKTTPPVLDSDGSFFLYSKLTVDKSRW QQGNVFSCSVMHEALHNHYTQKSLSLSPGK |
| Antibody-1 second heavy chain (SEQ ID NO. 3) | EVQLVESGGGLVQPGGSLRLSCAASGFTFTDYTMDWVRQAPGKGLE WVADVNPNSGGSILNRRFKGRFTLSVDRSKNTLYLQMNSLRAEDTAV YYCARNLGPLFYFDYWGQGTLVTVSSASTKGPSVFPLAPSSKSTSGG TAALGCLVKDYFPEPVTVSWNSGALTSGVHTFPAVLQSSGLYSLSSVV TVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVCTLPPSREEMTKNQVSLSCAVKGFYP SDIAVEWESNGQPENNYKTTPPVLDSDGSFFLVSKLTVDKSRWQQGN VFSCSVMHEALHNHYTQKSLSLSPGK |
| Human sialidase hNEU2-M (SEQ ID NO. 4) | DASLPYLQKESVFQSGAHAYRIPALLYLPGQQSLLAFAEQRRSKKDEH AELIVLRRGDYDAGTHQVQWQAQEVVAQARLDGHRSMNPCPLYDE QTGTLFLFFIAIPGQVTEQQQLQTRANVTRLCYVTSTDHGRTWSSPRD LTDAAIGPAYREWSTFAVGPGHCLQLHDRARSLVVPAYAYRKLHPKQ RPIPSAFCFLSHDHGRTWARGHFVAQDTLECQVAEVETGEQRVVTLN |
| | ARSHLRFRVQAQSTNDGLDFQESQLVKKLVEPPPTGCQGSVISFPSPR SGPGSPAQWLLYTHPTHSWQRADLGAYLNPRPPAPEAWSEPVLLAKG SAAYSDLQSMGTGPDGSPLFGCLYEANDYEEIVFLMFTLKQAFPAEY LPQ |
| Pongo sialidase pNEU2 (SEQ ID NO. 5) | MASLPVLQKESVFQSGAHAYRIPALLYLPGQQTLLAFAEQRASKKDE HAELIVLRRGDYDAPTHQVQWQAQEVVAQARLDGHRSMNPCPLYD AQTETLFLFFIAIPGQVTEQQQLQTRANVTRLCQVTSTDHGRTWSSPR DLTDAAIGPAYQEWSTFAVGPGHCLQLHDRARSLVVPAYAYRKLHPIQ RPSPSAFCFLSHDHGHTWVRGHFVAQDTLECQVAEVETGEQRVVTLN ARSHLQARVQAQSTNDGFDFQESQLVKKLVEPPPQGCQGSVISFPSPR SGPGSPAQWLLYTHPTHSWQRADLGAYLNPRPPAPEAWSEPVLLAKG SCAYSDLQSMGTGPDGSPLFGCLYEANDYEEIVFLMFTLKQAFPAEY LPQ |
| Hyaluronidase PH20 (SEQ ID NO. 6) | |
| Hyaluronidase HYAL1 (SEQ ID NO. 7) | FRGPLLPNRPFTTVWNANTQWCLERHGVDVDVSVFDVVANPGQTFR GPDMTIFYSSQLGTYPYYTPTGEPVFGGLPQNASLIAHLARTFQDILA AIPAPDFSGLAVIDWEAWRPRWAFNWDTKDIYRQRSRALVQAQHPD WPAPQVEAVAQDQFQGAARAWMAGTLQLGRALRPRGLWGFYGFPD CYNYDFLSPNYTGQCPSGIRAQNDQLGWLWGQSRALYPSIYMPAVLE GTGKSQMYVQHRVAEAFRVAVAAGDPNLPVLPYVQIFYDTTNHFLPL DELEHSLGESAAQGAAGVVLWVSWENTRTKESCQAIKEYMDTTLGP FILNVTSGALLCSQALCSGHGRCVRRTSHPKALLLLNPASFSIQLTPGG GPLSLRGALSLEDQAQMAVEFKCRCYPGWQAPWCERKSMW |
| Collagenase (SEQ ID NO. 8) | MKRKCLSKRLMLAITMATIFTVNSTLPIYAAVDKNNATAAVQNESKR YTVSYLKTLNYYDLVDLLVKTEIENLPDLFQYSSDAKEFYGNKTRMS FIMDEIGRRAPQYTEIDHKGIPTLVEVVRAGFYLGFHNKELNEINKRSF KERVIPSILAIQKNPNFKLGTEVQDKIVSATGLLAGNETAPPEVVNNFT PILQDCIKNIDRYALDDLKSKALFNVLAAPTYDITEYLRATKEKPENTP WYGKIDGFINELKKLALYGKINDNNSWIIDNGIYHIAPLGKLHSNNKI GIETLTEVMKVYPYLSMQHLQSADQIKRHYDSKDAEGNKIPLDKFKK EGKEKYCPKTYTFDDGKVIIKAGARVEEEKVKRLYWASKEVNSQFFR VYGIDKPLEEGNPDDILTMVIYNSPEEYKLNSVLYGYDTNNGGMYIEP EGTFFTYEREAQESTYTLEELFRHEYTHYLQGRYAVPGQWGRTKLYD NDRLTWYEEGGAELFAGSTRTSGILPRKSIVSNIHNTTRNNRYKLSDT VHSKYGASFEFYNYACMFMDYMYNKDMGILNKLNDLAKNNDVDG YDNYIRDLSSNYALNDKYQDHMQERIDNYENLTVPFVADDYLVRHA YKNPNEIYSEISEVAKLKDAKSEVKKSQYFSTFTLRGSYTGGASKGKL EDQKAMNKFIDDSLKKLDTYSWSGYKTLTAYFTNYKVDSSNRVTYD VVFHGYLPNEGDSKNSLPYGKINGTYKGTEKEKIKFSSEGSFDPDGKI VSYEWDFGDGNKSNEENPEHSYDKVGTYTVKLKVTDDKGESSVSTT TAEIKDLSENKLPVIYMHVPKSGALNQKVVFYGKGTYDPDGSIAGYQ WDFGDGSDFSSEQNPSHVYTKKGEYTVTLRVMDSSGQMSEKTMKIK ITDPVYPIGTEKEPNNSKETASGPIVPGIPVSGTIENTSDQDYFYFDVIT PGEVKIDINKLGYGGATWVVYDENNNAVSYATDDGQNLSGKFKADK PGRYYIHLYMFNGSYMPYRINIEGSVGR |
| Heparinase III (SEQ ID NO. 9) | MQSSSITRKDFDHINLEYSGLEKVNKAVAAGNYDDAAKALLAYYRE KSKAREPDFSNAEKPADIRQPIDKVTREMADKALVHQFQPHKGYGYF DYGKDINWQMWPVKDNEVRWQLHRVKWWQAMALVYHATGDEKY AREWVYQYSDWARKNPLGLSQDNDKFVWRPLEVSDRVQSLPPTFSL FVNSPAFTPAFLMEFLNSYHQQADYLSTHYAEQGNHRLFEAQRNLFA GVSFPEFKDSPRWRQTGISVLNTEIKKQVYADGMQFELSPIYHVAAIDI FLKAYGSAKRVNLEKEFPQSYVQTVENMIMALISISLPDYNTPMFGDS WITDKNFRMAQFASWARVFPANQAIKYFATDGKQGKAPNFLSKALSN AGFYTFRSGWDKNATVMVLKASPPGEFHAQPDNGTFELFIKGRNFTP DAGVFVYSGDEAIMKLRNWYRQTRIHSTLTLDNQNMVITKARQNK |
| | WETGNNLDVLTYTNPSYPNLDHQRSVLFINKKYFLVIDRAIGEATGNL GVHWQLKEDSNPVFDKTKNRVYTTYRDGNNLMIQSLNADRTSLNEE EGKVSYVVYNKELKRPAFVFEKPKKNAGTQNFVSIVYPYDGQKAPEISI RENKGNDFEKGKLNLTLTINGKQQLVLVPWSHPQFEK |
| Chondrosulphatase AC (SEQ ID NO. 10) | QQTGTAELIMKRVMLDLKKPLRNMDKVAEKDLNTLQPDGSWKDVP YKDDAMTNWLPNNHLLQLETIIQAYIEKDSHYYGDDKVFDQISKAFK YWYDSDPKSRNWWHNEIATPQALGEMLILMRYGKKPLDEALVHKLT ERMKRGEPEKKTGANKTDIALHYFYRALLTSDEALLSFAVKELFYPV QFVHYEEGLQYDYSYLQHGPQLQISSYGAVFITGVLKLANYVRDTPY ALSTEKLAIFSKYYRDSYLKAIRGSYMDFNVEGRGVSRPDILNKKAE KKRLLVAKMIDLKHTEEWADAIARTDSTVAAGYKIEPYHHQFWNGD YVQHLRPAYSFNVRMVSKRTRRSESGNKENLLGRYLSDGATNIQLRG PEYYNIMPVWEWDKIPGITSRDYLTDRPLTKLWGEQGSNDFAGGVSD GVYGASAYALDYDSLQAKKAWFFFDKEIVCLGAGINSNAPENITTTL NQSWLNGPVISTAGKTGRGKITTFKAQGQFWLLHDAIGYYFPEGANL SLSTQSQKGNWFHINNSHSKDEVSGDVFKLWINHGARPENAQYAYIV LPGINKPEEIKKYNGTAPKVLANTNQLQAVYHQQLDMVQAIFYTAGK LSVAGIEIETDKPCAVLIKHINGKQVIWAADPLQKEKTAVLSIRDLKTG KTNRVKIDFPQQEFAGATVELK |
| Chondroitinase (SEQ ID NO. 11) | MPIFRFTALAMTLGLLSAPYNAMAATSNPAFDPKNLMQSEIYHFAQN NPLADFSSDKNSILTLSDKRSIMGNQSLLWKWKGGSSFTLHKKLIVPT DKEASKAWGRSSTPVFSFWLYNEKPIDGYLTIDFGEKLISTSEAQAGF KVKLDFTGWRAVGVSLNNDLENREMTLNATNTSSDGTQDSIGRSLG AKVDSIRFKAPSNVSQGEIYIDRIMFSVDDARYQWSDYQVKTRLSEPE IQFHNVKPQLPVTPENLAAIDLIRQRLINEFVGGEKETNLALEENISKL KSDFDALNIHTLANGGTQGRHLITDKQIIIYQPENLNSQDKQLFDNYV ILGNYTTLMFNISRAYVLEKDPTQKAQLKQMYLLMTKHLLDQGFVK GSALVTTHHWGYSSRWWYISTLLMSDALKEANLQTQVYDSLLWYS REFKSSFDMKVSADSSDLDYFNTLSRQHLALLLLEPDDQKRINLVNTF SHYITGALTQVPPGGKDGLRPDGTAWRHEGNYPGYSFPAFKNASQLI YLLRDTPFSVGESGWNNLKKAMVSAWIYSNPEVGLPLAGRHPFNSPS LKSVAQGYYWLAMSAKSSPDKTLASIYLAISDKTQNESTAIFGETITP |
| | ASLPQGFYAFNGGAFGIHRWQDKMVTLKAYNTNVWSSEIYNKDNRY GRYQSHGVAQIVSNGSQLSQGYQQEGWDWNRMQGATTIHLPLKDLD SPKPHTLMQRGERGFSGTSSLEGQYGMMAFDLIYPANLERFDPNFTA KKSVLAADNHLIFIGSNINSSDKNKNVETTLFQHAITPTLNTLWINGQ KIENMPYQTTLQQGDWLIDSNGNGYLITQAEKVNVSRQHQVSAENK NRQPTEGNFSSAWIDHSTRPKDASYEYMVFLDATPEKMGEMAQKFR ENNGLYQVLRKDKDVHIILDKLSNVTGYAFYQPASIEDKWIKKVNKP AIVMTHRQKDTLIVSAVTPDLNMTRQKAATPVTINVTINGKWQSADK NSEVKYQVSGDNTELTFTSYFGIPQEIKLSPLP |
| Anti-Fc antibody (VHH Fragment) anFcVH (SEQ ID NO. 12) | QVQLVESGGGLVQAGGSLNLSCAVSDIMFSWSTMGWYRQAPGKERE LVAQERGSGRINYADFVKGRFTISRDSAKNTMNLQMNSLKPEDTGVY YCWVRRPSGSWRGQHYPDAQEYWGQGTQVTVSS |
| FcRn (SEQ ID NO. 13) | MGVPRPQPWALGLLLFLLPGSLGAESHLSLLYHLTAVSSPAPGTPAFW VSGWLGPQQYLSYNSLRGEAEPCGAWVWENQVSWYWEKETTDLRI KEKLFLEAFKALGGKGPYTLQGLLGCELGPDNTSVPTAKFALNGEEF MNFDLKQGTWGGDWPEALAISQRWQQQDKAANKELTFLLFSCPHR LREHLERGRGNLEWKEPPSMRLKARPSSPGFSVLTCSAFSFYPPELQL RFLRNGLAAGTGQGDFGPNSDGSFHASSSLTVKSGDEHHYCCIVQHA GLAQPLRVELESPAKSSVLVVGIVIGVLLLTAAVGGALLWRRMRSGL PAPWISLRGDDTGVLLPTPGEAQDADLKDVNVIPATA |
| Protein A (SEQ ID NO. 14) | MKKKNIYSIRKLGVGIASVTLGTLLISGGVTPAANAAQHDEAQQNAF YQVLNMPNLNADQRNGFIQSLKDDPSQSANVLGEAQKLNDSQAPKA DAQQNKFNKDQQSAFYEILNMPNLNEEQRNGFIQSLKDDPSQSTNVL GEAKKLNESQAPKADNNFNKEQQNAFYEILNMPNLNEEQRNGFIQSL KDDPSQSANLLAEAKKLNESQAPKADNKFNKEQQNAFYEILHLPNL NEEQRNGFIQSLKDDPSQSANLLAEAKKLNDAQAPKADNKFNKEQQ NAFYEILHLPNLTEEQRNGFIQSLKDDPSVSKEILAEAKKLNDAQAPK EEDNNKPGKEDGNKPGKEDGNKPGKEDNKKPGKEDGNKPGKEDNK KPGKEDGNKPGKEDGNKPGKEDGNKPGKEDGNKPGKEDGNGVHV VKPGDTVNDIAKANGTTADKIAADNKLADKNMIKPGQELVVDKKQP ANHADANKAQALPETGEENPFIGTTVFGGLSLALGAALLAGRRREL |
| Protein G (SEQ ID NO. 15) | MEKEKKVKYFLRKSAFGLASVSAAFLVGSTVFAVDSPIEDTPIIRNGG ELTNLLGNSETTLALRNEESATADLTAAAVADTVAAAAAENAGAAAW EAAAAADALAKAKADALKEFNKYGVSDYYKNLINNAKTVEGVKDL QAQVVESAKKARISEATDGLSDFLKSQTPAEDTVKSIELAEAKVLAN RELDKYGVSDYHKNLINNAKTVEGVKDLQAQVVESAKKARISEATD GLSDFLKSQTPAEDTVKSIELAEAKVLANRELDKYGVSDYYKNLINN AKTVEGVKALIDEILAALPKTDTYKLILNGKTLKGETTTEAVDAATAE KVFKQYANDNGVDGEWTYDDATKTFTVTEKPEVIDASELTPAVTTYK LVINGKTLKGETTTEAVDAATAEKVFKQYANDNGVDGEWTYDDATK TFTVTEKPEVIDASELTPAVTTYKLVINGKTLKGETTTKAVDAETAEK AFKQYANDNGVDGVWTYDDATKTFTVTEMVTEVPGDAPTEPEKPE ASIPLVPLTPATPIAKDDAKKDDTKKEDAKKPEAKKEDAKKAETLPTT GEGSNPFFTAAALAVMAGAGALAVASKRKED |
| Fc-binding peptide 1 (SEQ ID NO. 16) | EPIHRSTLTALL |
| Fc-binding peptide 2 (SEQ ID NO. 17) | TWKTSRISIF |
| Z33 (SEQ ID NO. 18) | FNMQQQRRFYEALHDPNLNEEQRNAKIKSIRDD |
| HYAL1-anFcVH (SEQ ID NO. 19) | FRGPLLPNRPFTTVWNANTQWCLERHGVDVDVSVFDVVANPGQTFR GPDMTIFYSSQLGTYPYYTPTGEPVFGGLPQNASLIAHLARTFQDILA AIPAPDFSGLAVIDWEAWRPRWAFNWDTKDIYRQRSRALVQAQHPD WPAPQVEAVAQDQFQGAARAWMAGTLQLGRALRPRGLWGFYGFPD CYNYDFLSPNYTGQCPSGIRAQNDQLGWLWGQSRALYPSIYMPAVLE GTGKSQMYVQHRVAEAFRVAVAAGDPNLPVLPYVQIFYDTTNHFLPL DELEHSLGESAAQGAAGVVLWVSWENTRTKESCQAIKEYMDTTLGP FILNVTSGALLCSQALCSGHGRCVRRTSHPKALLLLNPASFSIQLTPGG GPLSLRGALSLEDQAQMAVEFKCRCYPGWQAPWCERKSMWGGGGS GGGGSGGGGSGGGGSGGGGSQVQLVESGGGLVQAGGSLNLSCAVSD IMFSWSTMGWYRQAPGKERELVAQERGSGRINYADFVKGRFTISRDS AKNTMNLQMNSLKPEDTGVYYCWVRRPSGSWRGQHYPDAQEYWG QGTQVTVSS |
| PH20-anFcVH (SEQ ID NO. 20) | LNFRAPPVIPNVPFLWAWNAPSEFCLGKFDEPLDMSLFSFIGSPRINAT GQGVTIFYVDRLGYYPYIDSITGVTVNGGIPQKISLQDHLDKAKKDIT FYMPVDNLGMAVIDWEEWRPTWARNWKPKDVYKNRSIELVQQQNV QLSLTEATEKAKQEFEKAGKDFLVETIKLGKLLRPNHLWGYYLFPDC YNHHYKKPGYNGSCFNVEIKRNDDLSWLWNESTALYPSIYLNTQQSP VAATLYVRNRVREAIRVSKIPDAKSPLPVFAYTRIVFTDQVLKFLSQDE LVYTFGETVALGASGIVIWGTLSIMRSMKSCLLLDNYMETILNPYIINV TLAAKMCSQVLCQEQGVCIRKNWNSSDYLHLNPDNFAIQLEKGGKF TVRGKPTLEDLEQFSEKFYCSCYSTLSCKEKADVKDTDAVDVCIADG VCIDAFLKPPMETEEPQIFYGGGGSGGGGSGGGGSGGGGSGGGGSQ VQLVESGGGLVQAGGSLNLSCAVSDIMFSWSTMGWYRQAPGKEREL VAQERGSGRINYADFVKGRFTISRDSAKNTMNLQMNSLKPEDTGVY YCWVRRPSGSWRGQHYPDAQEYWGQGTQVTVSS |
| anFcVH-HYAL1 (SEQ ID NO. 21) | QVQLVESGGGLVQAGGSLNLSCAVSDIMFSWSTMGWYRQAPGKERE LVAQERGSGRINYADFVKGRFTISRDSAKNTMNLQMNSLKPEDTGVY YCWVRRPSGSWRGQHYPDAQEYWGQGTQVTVSSGGGGSGGGGSG GGGSGGGGSGGGGSFRGPLLPNRPFTTVWNANTQWCLERHGVDVD VSVFDVVANPGQTFRGPDMTIFYSSQLGTYPYYTPTGEPVFGGLPQN ASLIAHLARTFQDILAAIPAPDFSGLAVIDWEAWRPRWAFNWDTKDIY RQRSRALVQAQHPDWPAPQVEAVAQDQFQGAARAWMAGTLQLGRA LRPRGLWGFYGFPDCYNYDFLSPNYTGQCPSGIRAQNDQLGWLWGQ SRALYPSIYMPAVLEGTGKSQMYVQHRVAEAFRVAVAAGDPNLPVLP YVQIFYDTTNHFLPLDELEHSLGESAAQGAAGVVLWVSWENTRTKE SCQAIKEYMDTTLGPFILNVTSGALLCSQALCSGHGRCVRRTSHPKA LLLLNPASFSIQLTPGGGPLSLRGALSLEDQAQMAVEFKCRCYPGWQ APWCERKSMW |
| anFcVH-PH20 (SEQ ID NO. 22) | QVQLVESGGGLVQAGGSLNLSCAVSDIMFSWSTMGWYRQAPGKERE LVAQERGSGRINYADFVKGRFTISRDSAKNTMNLQMNSLKPEDTGVY YCWVRRPSGSWRGQHYPDAQEYWGQGTQVTVSSGGGGSGGGGSG GGGSGGGGSGGGGSLNFRAPPVIPNVPFLWAWNAPSEFCLGKFDEPL DMSLFSFIGSPRINATGQGVTIFYVDRLGYYPYIDSITGVTVNGGIPQKI SLQDHLDKAKKDITFYMPVDNLGMAVIDWEEWRPTWARNWKPKDV |
| | YKNRSIELVQQQNVQLSLTEATEKAKQEFEKAGKDFLVETIKLGKLLR PNHLWGYYLFPDCYNHHYKKPGYNGSCFNVEIKRNDDLSWLWNEST ALYPSIYLNTQQSPVAATLYVRNRVREAIRVSKIPDAKSPLPVFAYTRIV FTDQVLKFLSQDELVYTFGETVALGASGIVIWGTLSIMRSMKSCLLLD NYMETILNPYIINVTLAAKMCSQVLCQEQGVCIRKNWNSSDYLHLNP DNFAIQLEKGGKFTVRGKPTLEDLEQFSEKFYCSCYSTLSCKEKADV KDTDAVDVCIADGVCIDAFLKPPMETEEPQIFY |
| Collagenase-Protei n G (SEQ ID NO. 23) | MKRKCLSKRLMLAITMATIFTVNSTLPIYAAVDKNNATAAVQNESKR YTVSYLKTLNYYDLVDLLVKTEIENLPDLFQYSSDAKEFYGNKTRMS FIMDEIGRRAPQYTEIDHKGIPTLVEVVRAGFYLGFHNKELNEINKRSF KERVIPSILAIQKNPNFKLGTEVQDKIVSATGLLAGNETAPPEVVNNFT PILQDCIKNIDRYALDDLKSKALFNVLAAPTYDITEYLRATKEKPENTP WYGKIDGFINELKKLALYGKINDNNSWIIDNGIYHIAPLGKLHSNNKI GIETLTEVMKVYPYLSMQHLQSADQIKRHYDSKDAEGNKIPLDKFKK EGKEKYCPKTYTFDDGKVIIKAGARVEEEKVKRLYWASKEVNSQFFR VYGIDKPLEEGNPDDILTMVIYNSPEEYKLNSVLYGYDTNNGGMYIEP EGTFFTYEREAQESTYTLEELFRHEYTHYLQGRYAVPGQWGRTKLYD NDRLTWYEEGGAELFAGSTRTSGILPRKSIVSNIHNTTRNNRYKLSDT VHSKYGASFEFYNYACMFMDYMYNKDMGILNKLNDLAKNNDVDG YDNYIRDLSSNYALNDKYQDHMQERIDNYENLTVPFVADDYLVRHA YKNPNEIYSEISEVAKLKDAKSEVKKSQYFSTFTLRGSYTGGASKGKL EDQKAMNKFIDDSLKKLDTYSWSGYKTLTAYFTNYKVDSSNRVTYD VVFHGYLPNEGDSKNSLPYGKINGTYKGTEKEKIKFSSEGSFDPDGKI VSYEWDFGDGNKSNEENPEHSYDKVGTYTVKLKVTDDKGESSVSTT TAEIKDLSENKLPVIYMHVPKSGALNQKVVFYGKGTYDPDGSIAGYQ WDFGDGSDFSSEQNPSHVYTKKGEYTVTLRVMDSSGQMSEKTMKIK ITDPVYPIGTEKEPNNSKETASGPIVPGIPVSGTIENTSDQDYFYFDVIT PGEVKIDINKLGYGGATWVVYDENNNAVSYATDDGQNLSGKFKADK PGRYYIHLYMFNGSYMPYRINIEGSVGRGGGGSGGGGSGGGGSGGG GSGGGGSVDSPIEDTPIIRNGGELTNLLGNSETTLALRNEESATADLTA AAVADTVAAAAAENAGAAAWEAAAAADALAKAKADALKEFNKYG VSDYYKNLINNAKTVEGVKDLQAQVVESAKKARISEATDGLSDFLKS |
| | QTPAEDTVKSIELAEAKVLANRELDKYGVSDYHKNLINNAKTVEGV KDLQAQVVESAKKARISEATDGLSDFLKSQTPAEDTVKSIELAEAKVL ANRELDKYGVSDYYKNLINNAKTVEGVKALIDEILAALPKTDTYKLI LNGKTLKGETTTEAVDAATAEKVFKQYANDNGVDGEWTYDDATKTF TVTEKPEVIDASELTPAVTTYKLVINGKTLKGETTTEAVDAATAEKVF KQYANDNGVDGEWTYDDATKTFTVTEKPEVIDASELTPAVTTYKLVI NGKTLKGETTTKAVDAETAEKAFKQYANDNGVDGVWTYDDATKTF TVTEMVTEVPGDAPTEPEKPEASIPLVPLTPATPIAKDDAKKDDTKKE DAKKPEAKKEDAKKAETLPTTGEGSNPFFTAAALAVMAGAGALAVA SKRKED |
| Heparinase III-FcRn (SEQ ID NO. 24) | QSSSITRKDFDHINLEYSGLEKVNKAVAAGNYDDAAKALLAYYREKS KAREPDFSNAEKPADIRQPIDKVTREMADKALVHQFQPHKGYGYFD YGKDINWQMWPVKDNEVRWQLHRVKWWQAMALVYHATGDEKYA REWVYQYSDWARKNPLGLSQDNDKFVWRPLEVSDRVQSLPPTFSLF VNSPAFTPAFLMEFLNSYHQQADYLSTHYAEQGNHRLFEAQRNLFAG VSFPEFKDSPRWRQTGISVLNTEIKKQVYADGMQFELSPIYHVAAIDIF LKAYGSAKRVNLEKEFPQSYVQTVENMIMALISISLPDYNTPMFGDS WITDKNFRMAQFASWARVFPANQAIKYFATDGKQGKAPNFLSKALSN AGFYTFRSGWDKNATVMVLKASPPGEFHAQPDNGTFELFIKGRNFTP DAGVFVYSGDEAIMKLRNWYRQTRIHSTLTLDNQNMVITKARQNK WETGNNLDVLTYTNPSYPNLDHQRSVLFINKKYFLVIDRAIGEATGNL GVHWQLKEDSNPVFDKTKNRVYTTYRDGNNLMIQSLNADRTSLNEE EGKVSYVVYNKELKRPAFVFEKPKKNAGTQNFVSIVYPYDGQKAPEISI RENKGNDFEKGKLNLTLTINGKQQLVLVPWSHPQFEKGGGGSGGGG SGGGGSGGGGSGGGGSmgvprpqpwalglllfllpgslgaeshlsllyhltavsspapgtpaf wvsgwlgpqqylsynslrgeaepcgawvwenqvswywekettdlrikeklfleafkalggkgpytlqg llgcelgpdntsvptakfalngeefmnfdlkqgtwggdwpealaisqrwqqqdkaankeltfllfscphrl rehlergrgnlewkeppsmrlkarpsspgfsvltcsafsfyppelqlrflrnglaagtgqgdfgpnsdgsfh assslltvksgdehhyccivqhaglaqplrvelespakssvlvvgivigvllltaaavggallwrrmrsglpa pwislrgddtgvllptpgeaqdadlkdvnvipata |
| Chondroitinase-Z3 3 (SEQ ID NO. 25) | MPIFRFTALAMTLGLLSAPYNAMAATSNPAFDPKNLMQSEIYHFAQN NPLADFSSDKNSILTLSDKRSIMGNQSLLWKWKGGSSFTLHKKLIVPT |
| | DKEASKAWGRSSTPVFSFWLYNEKPIDGYLTIDFGEKLISTSEAQAGF KVKLDFTGWRAVGVSLNNDLENREMTLNATNTSSDGTQDSIGRSLG AKVDSIRFKAPSNVSQGEIYIDRIMFSVDDARYQWSDYQVKTRLSEPE IQFHNVKPQLPVTPENLAAIDLIRQRLINEFVGGEKETNLALEENISKL KSDFDALNIHTLANGGTQGRHLITDKQIIIYQPENLNSQDKQLFDNYV ILGNYTTLMFNISRAYVLEKDPTQKAQLKQMYLLMTKHLLDQGFVK GSALVTTHHWGYSSRWWYISTLLMSDALKEANLQTQVYDSLLWYS REFKSSFDMKVSADSSDLDYFNTLSRQHLALLLLEPDDQKRINLVNTF SHYITGALTQVPPGGKDGLRPDGTAWRHEGNYPGYSFPAFKNASQLI YLLRDTPFSVGESGWNNLKKAMVSAWIYSNPEVGLPLAGRHPFNSPS LKSVAQGYYWLAMSAKSSPDKTLASIYLAISDKTQNESTAIFGETITP ASLPQGFYAFNGGAFGIHRWQDKMVTLKAYNTNVWSSEIYNKDNRY GRYQSHGVAQIVSNGSQLSQGYQQEGWDWNRMQGATTIHLPLKDLD SPKPHTLMQRGERGFSGTSSLEGQYGMMAFDLIYPANLERFDPNFTA KKSVLAADNHLIFIGSNINSSDKNKNVETTLFQHAITPTLNTLWINGQ KIENMPYQTTLQQGDWLIDSNGNGYLITQAEKVNVSRQHQVSAENK NRQPTEGNFSSAWIDHSTRPKDASYEYMVFLDATPEKMGEMAQKFR ENNGLYQVLRKDKDVHIILDKLSNVTGYAFYQPASIEDKWIKKVNKP AIVMTHRQKDTLIVSAVTPDLNMTRQKAATPVTINVTINGKWQSADK NSEVKYQVSGDNTELTFTSYFGIPQEIKLSPLPGGGGSGGGGSGGGGS GGGGSGGGGSFNMQQQRRFYEALHDPNLNEEQRNAKIKSIRDD |
| Chondrosulphatase AC-Protein G (SEQ ID NO. 26) | QQTGTAELIMKRVMLDLKKPLRNMDKVAEKDLNTLQPDGSWKDVP YKDDAMTNWLPNNHLLQLETIIQAYIEKDSHYYGDDKVFDQISKAFK YWYDSDPKSRNWWHNEIATPQALGEMLILMRYGKKPLDEALVHKLT ERMKRGEPEKKTGANKTDIALHYFYRALLTSDEALLSFAVKELFYPV QFVHYEEGLQYDYSYLQHGPQLQISSYGAVFITGVLKLANYVRDTPY ALSTEKLAIFSKYYRDSYLKAIRGSYMDFNVEGRGVSRPDILNKKAE KKRLLVAKMIDLKHTEEWADAIARTDSTVAAGYKIEPYHHQFWNGD YVQHLRPAYSFNVRMVSKRTRRSESGNKENLLGRYLSDGATNIQLRG PEYYNIMPVWEWDKIPGITSRDYLTDRPLTKLWGEQGSNDFAGGVSD GVYGASAYALDYDSLQAKKAWFFFDKEIVCLGAGINSNAPENITTTL NQSWLNGPVISTAGKTGRGKITTFKAQGQFWLLHDAIGYYFPEGANL |
| | SLSTQSQKGNWFHINNSHSKDEVSGDVFKLWINHGARPENAQYAYIV LPGINKPEEIKKYNGTAPKVLANTNQLQAVYHQQLDMVQAIFYTAGK LSVAGIEIETDKPCAVLIKHINGKQVIWAADPLQKEKTAVLSIRDLKTG KTNRVKIDFPQQEFAGATVELKGGGGSGGGGSGGGGSGGGGSVDSPI EDTPIIRNGGELTNLLGNSETTLALRNEESATADLTAAAVADTVAAAA AENAGAAAWEAAAAADALAKAKADALKEFNKYGVSDYYKNLINN AKTVEGVKDLQAQVVESAKKARISEATDGLSDFLKSQTPAEDTVKSI ELAEAKVLANRELDKYGVSDYHKNLINNAKTVEGVKDLQAQVVES AKKARISEATDGLSDFLKSQTPAEDTVKSIELAEAKVLANRELDKYG VSDYYKNLINNAKTVEGVKALIDEILAALPKTDTYKLILNGKTLKGE TTTEAVDAATAEKVFKQYANDNGVDGEWTYDDATKTFTVTEKPEVI DASELTPAVTTYKLVINGKTLKGETTTEAVDAATAEKVFKQYANDNG VDGEWTYDDATKTFTVTEKPEVIDASELTPAVTTYKLVINGKTLKGET TTKAVDAETAEKAFKQYANDNGVDGVWTYDDATKTFTVTEMVTEV PGDAPTEPEKPEASIPLVPLTPATPIAKDDAKKDDTKKEDAKKPEAKK EDAKKAETLPTTGEGSNPFFTAAALAVMAGAGALAVASKRKED |
| hNEU2-M-Z33 (SEQ ID NO. 27) | DASLPYLQKESVFQSGAHAYRIPALLYLPGQQSLLAFAEQRRSKKDEH AELIVLRRGDYDAGTHQVQWQAQEVVAQARLDGHRSMNPCPLYDE QTGTLFLFFIAIPGQVTEQQQLQTRANVTRLCYVTSTDHGRTWSSPRD LTDAAIGPAYREWSTFAVGPGHCLQLHDRARSLVVPAYAYRKLHPKQ RPIPSAFCFLSHDHGRTWARGHFVAQDTLECQVAEVETGEQRVVTLN ARSHLRFRVQAQSTNDGLDFQESQLVKKLVEPPPTGCQGSVISFPSPR SGPGSPAQWLLYTHPTHSWQRADLGAYLNPRPPAPEAWSEPVLLAKG SAAYSDLQSMGTGPDGSPLFGCLYEANDYEEIVFLMFTLKQAFPAEY LPQGGGGSGGGGSGGGGSGGGGSFNMQQQRRFYEALHDPNLNEEQ RNAKIKSIRDD |
| Z33-hNEU2-M (SEQ ID NO. 28) | FNMQQQRRFYEALHDPNLNEEQRNAKIKSIRDDGGGGSGGGGSGGG GSGGGGSDASLPYLQKESVFQSGAHAYRIPALLYLPGQQSLLAFAEQR RSKKDEHAELIVLRRGDYDAGTHQVQWQAQEVVAQARLDGHRSMN PCPLYDEQTGTLFLFFIAIPGQVTEQQQLQTRANVTRLCYVTSTDHGR TWSSPRDLTDAAIGPAYREWSTFAVGPGHCLQLHDRARSLVVPAYAY RKLHPKQRPIPSAFCFLSHDHGRTWARGHFVAQDTLECQVAEVETGE |
| | QRVVTLNARSHLRFRVQAQSTNDGLDFQESQLVKKLVEPPPTGCQGS VISFPSPRSGPGSPAQWLLYTHPTHSWQRADLGAYLNPRPPAPEAWSE PVLLAKGSAAYSDLQSMGTGPDGSPLFGCLYEANDYEEIVFLMFTLK QAFPAEYLPQ |
| NEU2-M-anFcVH (SEQ ID NO. 29) | DASLPYLQKESVFQSGAHAYRIPALLYLPGQQSLLAFAEQRRSKKDEH AELIVLRRGDYDAGTHQVQWQAQEVVAQARLDGHRSMNPCPLYDE QTGTLFLFFIAIPGQVTEQQQLQTRANVTRLCYVTSTDHGRTWSSPRD LTDAAIGPAYREWSTFAVGPGHCLQLHDRARSLVVPAYAYRKLHPKQ RPIPSAFCFLSHDHGRTWARGHFVAQDTLECQVAEVETGEQRVVTLN ARSHLRFRVQAQSTNDGLDFQESQLVKKLVEPPPTGCQGSVISFPSPR SGPGSPAQWLLYTHPTHSWQRADLGAYLNPRPPAPEAWSEPVLLAKG SAAYSDLQSMGTGPDGSPLFGCLYEANDYEEIVFLMFTLKQAFPAEY LPQGGGGSGGGGSGGGGSGGGGSGGGGSQVQLVESGGGLVQAGGS LNLSCAVSDIMFSWSTMGWYRQAPGKERELVAQERGSGRINYADFV KGRFTISRDSAKNTMNLQMNSLKPEDTGVYYCWVRRPSGSWRGQH YPDAQEYWGQGTQVTVSS |
| anFcVH-NEU2-M (SEQ ID NO. 30) | QVQLVESGGGLVQAGGSLNLSCAVSDIMFSWSTMGWYRQAPGKERE LVAQERGSGRINYADFVKGRFTISRDSAKNTMNLQMNSLKPEDTGVY YCWVRRPSGSWRGQHYPDAQEYWGQGTQVTVSSGGGGSGGGGSG GGGSGGGGSGGGGSDASLPYLQKESVFQSGAHAYRIPALLYLPGQQS LLAFAEQRRSKKDEHAELIVLRRGDYDAGTHQVQWQAQEVVAQAR LDGHRSMNPCPLYDEQTGTLFLFFIAIPGQVTEQQQLQTRANVTRLC YVTSTDHGRTWSSPRDLTDAAIGPAYREWSTFAVGPGHCLQLHDRAR SLVVPAYAYRKLHPKQRPIPSAFCFLSHDHGRTWARGHFVAQDTLECQ VAEVETGEQRVVTLNARSHLRFRVQAQSTNDGLDFQESQLVKKLVEP PPTGCQGSVISFPSPRSGPGSPAQWLLYTHPTHSWQRADLGAYLNPRP PAPEAWSEPVLLAKGSAAYSDLQSMGTGPDGSPLFGCLYEANDYEEI VFLMFTLKQAFPAEYLPQ |
| anFcVH Kabat CDR1 (SEQ ID NO. 31) | WSTMG |
| anFcVH Kabat CDR2 (SEQ ID NO. 32) | QERGSGRINYADFVKG |
| anFcVH Kabat CDR3 (SEQ ID NO. 33) | RRPSGSWRGQHYPDAQEY |
| Z33 Q4L S29K (SEQ ID NO. 34) | FNMLQQRRFYEALHDPNLNEEQRNAKIKKIRDD |
| Z33 F1L Q4L (SEQ ID NO. 35) | LNMLQQRRFYEALHDPNLNEEQRNAKIKSIRDD |
| Z33 Q4L S29Q (SEQ ID NO. 36) | FNMLQQRRFYEALHDPNLNEEQRNAKIKQIRDD |
| Z33 F1L S29Q (SEQ ID NO. 37) | LNMQQQRRFYEALHDPNLNEEQRNAKIKQIRDD |
| Z33 Q4L (SEQ ID NO. 38) | FNMLQQRRFYEALHDPNLNEEQRNAKIKSIRDD |
| Z33 S29Q (SEQ ID NO. 39) | FNMQQQRRFYEALHDPNLNEEQRNAKIKQIRDD |
| Z33 Q6W S29K (SEQ ID NO. 40) | FNMQQWRRFYEALHDPNLNEEQRNAKIKKIRDD |
| Z33 F1L Q6W (SEQ ID NO. 41) | LNMQQWRRFYEALHDPNLNEEQRNAKIKSIRDD |
| LIGAND A (SEQ ID NO. 42) | EVQLLESGGGLVQPGGSLRLSCAASGFTFSSYAMSWVRQAPGKGLE WVSAISGSGGSTYYADSVKGRFTISRDNSKNTLYLQMNSLRAEDTAV YYCAKDFSSRRWYLEYWGQGTLVTVSSGGGGSGGGGSGGGGSGGG GSSSELTQDPAVSVALGQTVRITCQGDSLRSYYASWYQQKPGQAPVL VIYGKNNRPSGIPDRFSGSSSGNTASLTITGAQAEDEADYYCNSLERIG YLSYVFGGGTKLTVL |
| LIGAND A-Z33 Q4L (SEQ ID NO. 43) | EVQLLESGGGLVQPGGSLRLSCAASGFTFSSYAMSWVRQAPGKGLE WVSAISGSGGSTYYADSVKGRFTISRDNSKNTLYLQMNSLRAEDTAV YYCAKDFSSRRWYLEYWGQGTLVTVSSGGGGSGGGGSGGGGSGGG GSSSELTQDPAVSVALGQTVRITCQGDSLRSYYASWYQQKPGQAPVL VIYGKNNRPSGIPDRFSGSSSGNTASLTITGAQAEDEADYYCNSLERIG |
| | YLSYVFGGGTKLTVLGGGGSGGGGSGGGGSGGGGSGGGGSFNMLQ QRRFYEALHDPNLNEEQRNAKIKSIRDD |
| LIGAND A-Z33 F1L (SEQ ID NO. 44) | EVQLLESGGGLVQPGGSLRLSCAASGFTFSSYAMSWVRQAPGKGLE WVSAISGSGGSTYYADSVKGRFTISRDNSKNTLYLQMNSLRAEDTAV YYCAKDFSSRRWYLEYWGQGTLVTVSSGGGGSGGGGSGGGGSGGG GSSSELTQDPAVSVALGQTVRITCQGDSLRSYYASWYQQKPGQAPVL VIYGKNNRPSGIPDRFSGSSSGNTASLTITGAQAEDEADYYCNSLERIG YLSYVFGGGTKLTVLGGGGSGGGGSGGGGSGGGGSGGGGSLNMQQ QRRFYEALHDPNLNEEQRNAKIKSIRDD |
| LIGAND A-Z33 S29Q (SEQ ID NO. 45) | EVQLLESGGGLVQPGGSLRLSCAASGFTFSSYAMSWVRQAPGKGLE WVSAISGSGGSTYYADSVKGRFTISRDNSKNTLYLQMNSLRAEDTAV YYCAKDFSSRRWYLEYWGQGTLVTVSSGGGGSGGGGSGGGGSGGG GSSSELTQDPAVSVALGQTVRITCQGDSLRSYYASWYQQKPGQAPVL VIYGKNNRPSGIPDRFSGSSSGNTASLTITGAQAEDEADYYCNSLERIG YLSYVFGGGTKLTVLGGGGSGGGGSGGGGSGGGGSGGGGSFNMQQ QRRFYEALHDPNLNEEQRNAKIKQIRDD |
| LIGAND A-Z33 Q6W (SEQ ID NO. 46) | EVQLLESGGGLVQPGGSLRLSCAASGFTFSSYAMSWVRQAPGKGLE WVSAISGSGGSTYYADSVKGRFTISRDNSKNTLYLQMNSLRAEDTAV YYCAKDFSSRRWYLEYWGQGTLVTVSSGGGGSGGGGSGGGGSGGG GSSSELTQDPAVSVALGQTVRITCQGDSLRSYYASWYQQKPGQAPVL VIYGKNNRPSGIPDRFSGSSSGNTASLTITGAQAEDEADYYCNSLERIG YLSYVFGGGTKLTVLGGGGSGGGGSGGGGSGGGGSGGGGSFNMQQ WRRFYEALHDPNLNEEQRNAKIKSIRDD |
| LIGAND A-Z33 WT (SEQ ID NO. 47) | EVQLLESGGGLVQPGGSLRLSCAASGFTFSSYAMSWVRQAPGKGLE WVSAISGSGGSTYYADSVKGRFTISRDNSKNTLYLQMNSLRAEDTAV YYCAKDFSSRRWYLEYWGQGTLVTVSSGGGGSGGGGSGGGGSGGG GSSSELTQDPAVSVALGQTVRITCQGDSLRSYYASWYQQKPGQAPVL VIYGKNNRPSGIPDRFSGSSSGNTASLTITGAQAEDEADYYCNSLERIG YLSYVFGGGTKLTVLGGGGSGGGGSGGGGSGGGGSGGGGSFNMQQ QRRFYEALHDPNLNEEQRNAKIKSIRDD |
| LIGAND A-Z33 P16A-N17L-N18M (SEQ ID NO. 48) | EVQLLESGGGLVQPGGSLRLSCAASGFTFSSYAMSWVRQAPGKGLE WVSAISGSGGSTYYADSVKGRFTISRDNSKNTLYLQMNSLRAEDTAV |
| | YYCAKDFSSRRWYLEYWGQGTLVTVSSGGGGSGGGGSGGGGSGGG GSSSELTQDPAVSVALGQTVRITCQGDSLRSYYASWYQQKPGQAPVL VIYGKNNRPSGIPDRFSGSSSGNTASLTITGAQAEDEADYYCNSLERIG YLSYVFGGGTKLTVLGGGGSGGGGSGGGGSGGGGSGGGGSFNMQQ QRRFYEALHDALMNEEQRNAKIKSIRDD |
| LIGAND A-Z33 P16A-N17L-N18M -S29R (SEQ ID NO. 49) | EVQLLESGGGLVQPGGSLRLSCAASGFTFSSYAMSWVRQAPGKGLE WVSAISGSGGSTYYADSVKGRFTISRDNSKNTLYLQMNSLRAEDTAV YYCAKDFSSRRWYLEYWGQGTLVTVSSGGGGSGGGGSGGGGSGGG GSSSELTQDPAVSVALGQTVRITCQGDSLRSYYASWYQQKPGQAPVL VIYGKNNRPSGIPDRFSGSSSGNTASLTITGAQAEDEADYYCNSLERIG YLSYVFGGGTKLTVLGGGGSGGGGSGGGGSGGGGSGGGGSFNMQQ QRRFYEALHDALMNEEQRNAKIKRIRDD |
| LIGAND A-Z33 P16A-N17L-N18M -S29N (SEQ ID NO. 50) | EVQLLESGGGLVQPGGSLRLSCAASGFTFSSYAMSWVRQAPGKGLE WVSAISGSGGSTYYADSVKGRFTISRDNSKNTLYLQMNSLRAEDTAV YYCAKDFSSRRWYLEYWGQGTLVTVSSGGGGSGGGGSGGGGSGGG GSSSELTQDPAVSVALGQTVRITCQGDSLRSYYASWYQQKPGQAPVL VIYGKNNRPSGIPDRFSGSSSGNTASLTITGAQAEDEADYYCNSLERIG YLSYVFGGGTKLTVLGGGGSGGGGSGGGGSGGGGSGGGGSFNMQQ QRRFYEALHDALMNEEQRNAKIKNIRDD |
| LIGAND A-Z33 A12T-H14W-P16L -N17T (SEQ ID NO. 51) | EVQLLESGGGLVQPGGSLRLSCAASGFTFSSYAMSWVRQAPGKGLE WVSAISGSGGSTYYADSVKGRFTISRDNSKNTLYLQMNSLRAEDTAV YYCAKDFSSRRWYLEYWGQGTLVTVSSGGGGSGGGGSGGGGSGGG GSSSELTQDPAVSVALGQTVRITCQGDSLRSYYASWYQQKPGQAPVL VIYGKNNRPSGIPDRFSGSSSGNTASLTITGAQAEDEADYYCNSLERIG YLSYVFGGGTKLTVLGGGGSGGGGSGGGGSGGGGSGGGGSFNMQQ QRRFYETLWDLTLNEEQRNAKIKSIRDD |
| LIGAND A-Z33 A12T-H14W-P16L -N17T-S29K (SEQ ID NO. 52) | EVQLLESGGGLVQPGGSLRLSCAASGFTFSSYAMSWVRQAPGKGLE WVSAISGSGGSTYYADSVKGRFTISRDNSKNTLYLQMNSLRAEDTAV YYCAKDFSSRRWYLEYWGQGTLVTVSSGGGGSGGGGSGGGGSGGG GSSSELTQDPAVSVALGQTVRITCQGDSLRSYYASWYQQKPGQAPVL VIYGKNNRPSGIPDRFSGSSSGNTASLTITGAQAEDEADYYCNSLERIG YLSYVFGGGTKLTVLGGGGSGGGGSGGGGSGGGGSGGGGSFNMQQ |
| | QRRFYETLWDLTLNEEQRNAKIKKIRDD |
| LIGAND A-Z33 E11D-P16L-N17L (SEQ ID NO. 53) | EVQLLESGGGLVQPGGSLRLSCAASGFTFSSYAMSWVRQAPGKGLE WVSAISGSGGSTYYADSVKGRFTISRDNSKNTLYLQMNSLRAEDTAV YYCAKDFSSRRWYLEYWGQGTLVTVSSGGGGSGGGGSGGGGSGGG GSSSELTQDPAVSVALGQTVRITCQGDSLRSYYASWYQQKPGQAPVL VIYGKNNRPSGIPDRFSGSSSGNTASLTITGAQAEDEADYYCNSLERIG YLSYVFGGGTKLTVLGGGGSGGGGSGGGGSGGGGSGGGGSFNMQQ QRRFYDALHDLLLNEEQRNAKIKSIRDD |
| LIGAND A-Z33 Q4L-E11D-P16L-N17L (SEQ ID NO. 54) | EVQLLESGGGLVQPGGSLRLSCAASGFTFSSYAMSWVRQAPGKGLE WVSAISGSGGSTYYADSVKGRFTISRDNSKNTLYLQMNSLRAEDTAV YYCAKDFSSRRWYLEYWGQGTLVTVSSGGGGSGGGGSGGGGSGGG GSSSELTQDPAVSVALGQTVRITCQGDSLRSYYASWYQQKPGQAPVL VIYGKNNRPSGIPDRFSGSSSGNTASLTITGAQAEDEADYYCNSLERIG YLSYVFGGGTKLTVLGGGGSGGGGSGGGGSGGGGSGGGGSFNMLQ QRRFYDALHDLLLNEEQRNAKIKSIRDD |
| Human Neu1 (SEQ ID NO. 55) | ENDFGLVQPLVTMEQLLWVSGRQIGSVDTFRIPLITATPRGTLLAFAEA RKMSSSDEGAKFIALRRSMDQGSTWSPTAFIVNDGDVPDGLNLGAVV SDVETGVVFLFYSLCAHKAGCQVASTMLVWSKDDGVSWSTPRNLSL DIGTEVFAPGPGSGIQKQREPRKGRLIVCGHGTLERDGVFCLLSDDHG ASWRYGSGVSGIPYGQPKQENDFNPDECQPYELPDGSVVINARNQN NYHCHCRIVLRSYDACDTLRPRDVTFDPELVDPVVAAGAVVTSSGIV FFSNPAHPEFRVNLTLRWSFSNGTSWRKETVQLWPGPSGYSSLATLEG SMDGEEQAPQLYVLYEKGRNHYTESISVAKISV |
| Human Neu2 (SEQ ID NO. 56) | MASLPVLQKESVFQSGAHAYRIPALLYLPGQQSLLAFAEQRASKKDE HAELIVLRRGDYDAPTHQVQWQAQEVVAQARLDGHRSMNPCPLYD AQTGTLFLFFIAIPGQVTEQQQLQTRANVTRLCQVTSTDHGRTWSSPR DLTDAAIGPAYREWSTFAVGPGHCLQLHDRARSLVVPAYAYRKLHPIQ RPIPSAFCFLSHDHGRTWARGHFVAQDTLECQVAEVETGEQRVVTLN ARSHLRARVQAQSTNDGLDFQESQLVKKLVEPPPQGCQGSVISFPSPR SGPGSPAQWLLYTHPTHSWQRADLGAYLNPRPPAPEAWSEPVLLAKG SCAYSDLQSMGTGPDGSPLFGCLYEANDYEEIVFLMFTLKQAFPAEY LPQ |
| Human Neu3-1 (SEQ ID NO. 57) | MEEVTTCSFNSPLFRQEDDRGITYRIPALLYIPPTHTFLAFAEKRSTRR DEDALHLVLRRGLRIGQLVQWGPLKPLMEATLPGHRTMNPCPVWEQ KSGCVFLFFICVRGHVTERQQIVSGRNAARLCFIYSQDAGCSWSEVR DLTEEVIGSELKHWATFAVGPGHGIQLQSGRLVIPAYTYYIPSWFFCFQ LPCKTRPHSLMIYSDDLGVTWHHGRLIRPMVTVECEVAEVTGRAGHP VLYCSARTPNRCRAEALSTDHGEGFQRLALSRQLCEPPHGCQGSVVS FRPLEIPHRCQDSSSKDAPTIQQSSPGSSLRLEEEAGTPSESWLLYSHPT SRKQRVDLGIYLNQTPLEAACWSRPWILHCGPCGYSDLAALEEEGLF GCLFECGTKQECEQIAFRLFTHREILSHLQGDCTSPGRNPSQFKSN |
| Human Neu3-2 (SEQ ID NO. 58) | MRPADLPPRPMEESPASSSAPTETEEPGSSAEVMEEVTTCSFNSPLFRQ EDDRGITYRIPALLYIPPTHTFLAFAEKRSTRRDEDALHLVLRRGLRIG QLVQWGPLKPLMEATLPGHRTMNPCPVWEQKSGCVFLFFICVRGHV TERQQIVSGRNAARLCFIYSQDAGCSWSEVRDLTEEVIGSELKHWATF AVGPGHGIQLQSGRLVIPAYTYYIPSWFFCFQLPCKTRPHSLMIYSDDL GVTWHHGRLIRPMVTVECEVAEVTGRAGHPVLYCSARTPNRCRAEA LSTDHGEGFQRLALSRQLCEPPHGCQGSVVSFRPLEIPHRCQDSSSKD APTIQQSSPGSSLRLEEEAGTPSESWLLYSHPTSRKQRVDLGIYLNQTP LEAACWSRPWILHCGPCGYSDLAALEEEGLFGCLFECGTKQECEQIA FRLFTHREILSHLQGDCTSPGRNPSQFKSN |
| Human Neu4-1 (SEQ ID NO. 59) | MGVPRTPSRTVLFERERTGLTYRVPSLLPVPPGPTLLAFVEQRLSPDDS HAHRLVLRRGTLAGGSVRWGALHVLGTAALAEHRSMNPCPVHDAG TGTVFLFFIAVLGHTPEAVQIATGRNAARLCCVASRDAGLSWGSARDL TEEAIGGAVQDWATFAVGPGHGVQLPSGRLLVPAYTYRVDRRECFGKI CRTSPHSFAFYSDDHGRTWRCGGLVPNLRSGECQLAAVDGGQAGSFL YCNARSPLGSRVQALSTDEGTSFLPAERVASLPETAWGCQGSIVGFPAP APNRPRDDSWSVGPGSPLQPPLLGPGVHEPPEEAAVDPRGGQVPGGP FSRLQPRGDGPRQPGPRPGVSGDVGSWTLALPMPFAAPPQSPTWLLY SHPVGRRARLHMGIRLSQSPLDPRSWTEPWVIYEGPSGYSDLASIGPA PEGGLVFACLYESGARTSYDEISFCTFSLREVLENVPASPKPPNLGDKP RGCCWPS |
| Human Neu4-2 (SEQ ID NO. 60) | MMSSAAFPRWLSMGVPRTPSRTVLFERERTGLTYRVPSLLPVPPGPTL LAFVEQRLSPDDSHAHRLVLRRGTLAGGSVRWGALHVLGTAALAEH |
| | RSMNPCPVHDAGTGTVFLFFIAVLGHTPEAVQIATGRNAARLCCVASR DAGLSWGSARDLTEEAIGGAVQDWATFAVGPGHGVQLPSGRLLVPAY TYRVDRRECFGKICRTSPHSFAFYSDDHGRTWRCGGLVPNLRSGECQ LAAVDGGQAGSFLYCNARSPLGSRVQALSTDEGTSFLPAERVASLPET AWGCQGSIVGFPAPAPNRPRDDSWSVGPGSPLQPPLLGPGVHEPPEEA AVDPRGGQVPGGPFSRLQPRGDGPRQPGPRPGVSGDVGSWTLALPM PFAAPPQSPTWLLYSHPVGRRARLHMGIRLSQSPLDPRSWTEPWVIYE GPSGYSDLASIGPAPEGGLVFACLYESGARTSYDEISFCTFSLREVLEN VPASPKPPNLGDKPRGCCWPS |
| Z33-mut1-PH20 (SEQ ID NO. 61) | FNMQQQRRFYEALHDPNLNEEQRNAKIKQIRDDGGGGSGGGGSGGG GSGGGGSGGGGSLNFRAPPVIPNVPFLWAWNAPSEFCLGKFDEPLDM SLFSFIGSPRINATGQGVTIFYVDRLGYYPYIDSITGVTVNGGIPQKISL QDHLDKAKKDITFYMPVDNLGMAVIDWEEWRPTWARNWKPKDVY KNRSIELVQQQNVQLSLTEATEKAKQEFEKAGKDFLVETIKLGKLLRP NHLWGYYLFPDCYNHHYKKPGYNGSCFNVEIKRNDDLSWLWNEST ALYPSIYLNTQQSPVAATLYVRNRVREAIRVSKIPDAKSPLPVFAYTRIV FTDQVLKFLSQDELVYTFGETVALGASGIVIWGTLSIMRSMKSCLLLD NYMETILNPYIINVTLAAKMCSQVLCQEQGVCIRKNWNSSDYLHLNP DNFAIQLEKGGKFTVRGKPTLEDLEQFSEKFYCSCYSTLSCKEKADV KDTDAVDVCIADGVCIDAFLKPPMETEEPQIFY |
| PH20-Z33-mut1 (SEQ ID NO. 62) | LNFRAPPVIPNVPFLWAWNAPSEFCLGKFDEPLDMSLFSFIGSPRINAT GQGVTIFYVDRLGYYPYIDSITGVTVNGGIPQKISLQDHLDKAKKDIT FYMPVDNLGMAVIDWEEWRPTWARNWKPKDVYKNRSIELVQQQNV QLSLTEATEKAKQEFEKAGKDFLVETIKLGKLLRPNHLWGYYLFPDC YNHHYKKPGYNGSCFNVEIKRNDDLSWLWNESTALYPSIYLNTQQSP VAATLYVRNRVREAIRVSKIPDAKSPLPVFAYTRIVFTDQVLKFLSQDE LVYTFGETVALGASGIVIWGTLSIMRSMKSCLLLDNYMETILNPYIINV TLAAKMCSQVLCQEQGVCIRKNWNSSDYLHLNPDNFAIQLEKGGKF TVRGKPTLEDLEQFSEKFYCSCYSTLSCKEKADVKDTDAVDVCIADG VCIDAFLKPPMETEEPQIFYGGGGSGGGGSGGGGSGGGGSGGGGSFN MQQQRRFYEALHDPNLNEEQRNAKIKQIRDD |
| Z33-mut2-PH20 (SEQ ID NO. 63) | FNMLQQRRFYEALHDPNLNEEQRNAKIKSIRDDGGGGSGGGGSGGG |
| | GSGGGGSGGGGSLNFRAPPVIPNVPFLWAWNAPSEFCLGKFDEPLDM SLFSFIGSPRINATGQGVTIFYVDRLGYYPYIDSITGVTVNGGIPQKISL QDHLDKAKKDITFYMPVDNLGMAVIDWEEWRPTWARNWKPKDVY KNRSIELVQQQNVQLSLTEATEKAKQEFEKAGKDFLVETIKLGKLLRP NHLWGYYLFPDCYNHHYKKPGYNGSCFNVEIKRNDDLSWLWNEST ALYPSIYLNTQQSPVAATLYVRNRVREAIRVSKIPDAKSPLPVFAYTRIV FTDQVLKFLSQDELVYTFGETVALGASGIVIWGTLSIMRSMKSCLLLD NYMETILNPYIINVTLAAKMCSQVLCQEQGVCIRKNWNSSDYLHLNP DNFAIQLEKGGKFTVRGKPTLEDLEQFSEKFYCSCYSTLSCKEKADV KDTDAVDVCIADGVCIDAFLKPPMETEEPQIFY |
| PH20-Z33-mut2 (SEQ ID NO. 64) | LNFRAPPVIPNVPFLWAWNAPSEFCLGKFDEPLDMSLFSFIGSPRINAT GQGVTIFYVDRLGYYPYIDSITGVTVNGGIPQKISLQDHLDKAKKDIT FYMPVDNLGMAVIDWEEWRPTWARNWKPKDVYKNRSIELVQQQNV QLSLTEATEKAKQEFEKAGKDFLVETIKLGKLLRPNHLWGYYLFPDC YNHHYKKPGYNGSCFNVEIKRNDDLSWLWNESTALYPSIYLNTQQSP VAATLYVRNRVREAIRVSKIPDAKSPLPVFAYTRIVFTDQVLKFLSQDE LVYTFGETVALGASGIVIWGTLSIMRSMKSCLLLDNYMETILNPYIINV TLAAKMCSQVLCQEQGVCIRKNWNSSDYLHLNPDNFAIQLEKGGKF TVRGKPTLEDLEQFSEKFYCSCYSTLSCKEKADVKDTDAVDVCIADG VCIDAFLKPPMETEEPQIFYGGGGSGGGGSGGGGSGGGGSGGGGSFN MLQQRRFYEALHDPNLNEEQRNAKIKSIRDD |
| Hyaluronidase PH20-M1 (SEQ ID NO. 65) | LNFRAPPVIPNVPFLWAWNAPSEFCLGKFDEPLDMSLFSFIGSPRINAT GQGVTIFYVDRLGYYPYIDSITGVTVNGGIPQKISLQDHLDKAKKDIT FYMPVDNLGMAVIDWEEWRPTWARNWKPKDVYKNRSIELVQQQNV QLSLTEATEKAKQEFEKAGKDFLVETIKLGKLLRPNHLWGYYLFPDC YNHHYKKPGYNGSCFNVEIKRNDDLSWLWNESTALYPSIYLNTQQSP VAATLYVRNRVREAIRVSKIPDAKSPLPVFAYTRIVFTDQVLKFLSQDE LVYTFGETVALGASGIVIWGTLSIMRSMKSCLLLDNYMETILNPYIINV TLAAKMCSQVLCQEQGVCIRKNWNSSDYLHLNPDNFAIQLEKGGKF TVRGKPTLEDLEQFSEKFYCSCYSTLSCKEKADVKDTDAVDVCIADG VCIDAFLKPPMETEEPQIF |
| Hyaluronidase PH20-M2 (SEQ ID NO. 66) | LNFRAPPVIPNVPFLWAWNAPSEFCLGKFDEPLDMSLFSFIGSPRINAT |
| | GQGVTIFYVDRLGYYPYIDSITGVTVNGGIPQKISLQDHLDKAKKDIT FYMPVDNLGMAVIDWEEWRPTWARNWKPKDVYKNRSIELVQQQNV QLSLTEATEKAKQEFEKAGKDFLVETIKLGKLLRPNHLWGYYLFPDC YNHHYKKPGYNGSCFNVEIKRNDDLSWLWNESTALYPSIYLNTQQSP VAATLYVRNRVREAIRVSKIPDAKSPLPVFAYTRIVFTDQVLKFLSQDE LVYTFGETVALGASGIVIWGTLSIMRSMKSCLLLDNYMETILNPYIINV TLAAKMCSQVLCQEQGVCIRKNWNSSDYLHLNPDNFAIQLEKGGKF TVRGKPTLEDLEQFSEKFYCSCYSTLSCKEKADVKDTDAVDVCIADG VCIDAFLKPPMETEEPQI |
| Hyaluronidase PH20-M3 (SEQ ID NO. 67) | LNFRAPPVIPNVPFLWAWNAPSEFCLGKFDEPLDMSLFSFIGSPRINAT GQGVTIFYVDRLGYYPYIDSITGVTVNGGIPQKISLQDHLDKAKKDIT FYMPVDNLGMAVIDWEEWRPTWARNWKPKDVYKNRSIELVQQQNV QLSLTEATEKAKQEFEKAGKDFLVETIKLGKLLRPNHLWGYYLFPDC YNHHYKKPGYNGSCFNVEIKRNDDLSWLWNESTALYPSIYLNTQQSP VAATLYVRNRVREAIRVSKIPDAKSPLPVFAYTRIVFTDQVLKFLSQDE LVYTFGETVALGASGIVIWGTLSIMRSMKSCLLLDNYMETILNPYIINV TLAAKMCSQVLCQEQGVCIRKNWNSSDYLHLNPDNFAIQLEKGGKF TVRGKPTLEDLEQFSEKFYCSCYSTLSCKEKADVKDTDAVDVCIADG VCIDAFLKPPMETEEPQ |
| Hyaluronidase PH20-M4 (SEQ ID NO. 68) | LNFRAPPVIPNVPFLWAWNAPSEFCLGKFDEPLDMSLFSFIGSPRINAT GQGVTIFYVDRLGYYPYIDSITGVTVNGGIPQKISLQDHLDKAKKDIT FYMPVDNLGMAVIDWEEWRPTWARNWKPKDVYKNRSIELVQQQNV QLSLTEATEKAKQEFEKAGKDFLVETIKLGKLLRPNHLWGYYLFPDC YNHHYKKPGYNGSCFNVEIKRNDDLSWLWNESTALYPSIYLNTQQSP VAATLYVRNRVREAIRVSKIPDAKSPLPVFAYTRIVFTDQVLKFLSQDE LVYTFGETVALGASGIVIWGTLSIMRSMKSCLLLDNYMETILNPYIINV TLAAKMCSQVLCQEQGVCIRKNWNSSDYLHLNPDNFAIQLEKGGKF TVRGKPTLEDLEQFSEKFYCSCYSTLSCKEKADVKDTDAVDVCIADG VCIDAFLKPPMETEEP |
| Hyaluronidase PH20-M5 (SEQ ID NO. 69) | LNFRAPPVIPNVPFLWAWNAPSEFCLGKFDEPLDMSLFSFIGSPRINAT GQGVTIFYVDRLGYYPYIDSITGVTVNGGIPQKISLQDHLDKAKKDIT FYMPVDNLGMAVIDWEEWRPTWARNWKPKDVYKNRSIELVQQQNV |
| | QLSLTEATEKAKQEFEKAGKDFLVETIKLGKLLRPNHLWGYYLFPDC YNHHYKKPGYNGSCFNVEIKRNDDLSWLWNESTALYPSIYLNTQQSP VAATLYVRNRVREAIRVSKIPDAKSPLPVFAYTRIVFTDQVLKFLSQDE LVYTFGETVALGASGIVIWGTLSIMRSMKSCLLLDNYMETILNPYIINV TLAAKMCSQVLCQEQGVCIRKNWNSSDYLHLNPDNFAIQLEKGGKF TVRGKPTLEDLEQFSEKFYCSCYSTLSCKEKADVKDTDAVDVCIADG VCIDAFLKPPME |
| Hyaluronidase PH20-M6 (SEQ ID NO. 70) | LNFRAPPVIPNVPFLWAWNAPSEFCLGKFDEPLDMSLFSFIGSPRINAT GQGVTIFYVDRLGYYPYIDSITGVTVNGGIPQKISLQDHLDKAKKDIT FYMPVDNLGMAVIDWEEWRPTWARNWKPKDVYKNRSIELVQQQNV QLSLTEATEKAKQEFEKAGKDFLVETIKLGKLLRPNHLWGYYLFPDC YNHHYKKPGYNGSCFNVEIKRNDDLSWLWNESTALYPSIYLNTQQSP VAATLYVRNRVREAIRVSKIPDAKSPLPVFAYTRIVFTDQVLKFLSQDE LVYTFGETVALGASGIVIWGTLSIMRSMKSCLLLDNYMETILNPYIINV TLAAKMCSQVLCQEQGVCIRKNWNSSDYLHLNPDNFAIQLEKGGKF TVRGKPTLEDLEQFSEKFYCSCYSTLSCKEKADVKDTDAVDVCIADG VCIDAFLKPPM |
| Hyaluronidase PH20-M7 (SEQ ID NO. 71) | FRAPPVIPNVPFLWAWNAPSEFCLGKFDEPLDMSLFSFIGSPRINATGQ GVTIFYVDRLGYYPYIDSITGVTVNGGIPQKISLQDHLDKAKKDITFY MPVDNLGMAVIDWEEWRPTWARNWKPKDVYKNRSIELVQQQNVQL SLTEATEKAKQEFEKAGKDFLVETIKLGKLLRPNHLWGYYLFPDCYN HHYKKPGYNGSCFNVEIKRNDDLSWLWNESTALYPSIYLNTQQSPVA ATLYVRNRVREAIRVSKIPDAKSPLPVFAYTRIVFTDQVLKFLSQDELV YTFGETVALGASGIVIWGSWENTRTKESCQAIKEYMDTTLNPYIINVT LAAKMCSQVLCQEQGVCIRKNWNSSDYLHLNPDNFAIQLEKGGKFT VRGKPTLEDLEQFSEKFYCSCYSTLSCKEKADVKDTDAVDVCIADGV CIDAF |

### Example 2: Enzymatic Activity Detection of Human Sialidase NEU2-M-anFcVH Fusion Protein (SEQ ID NO. 29)

The purified NEU2-M-anFcVH was diluted to 100 µg/ml with PBS, and then serially diluted 3-fold for 5 times, with PBS as a blank control. The enzyme cleavage substrate 4-MU-NANA was diluted to 2 mM and mixed with NEU2-M-anFcVH at a 1:1 volume ratio. After incubation at 37°C, the reaction was terminated by adding 0.1 M Na₂CO₃ at pH 10.5. The fluorescence values of λ_{cx} at 365 nm and λ_{cm} at 450 nm were detected using a microplate reader. The higher the fluorescence value, the higher the enzymatic activity. After subtracting the fluorescence value of the blank control from the fluorescence values of the samples, the fluorescence values were fitted with a four-parameter curve, and the EC₅₀ was calculated.

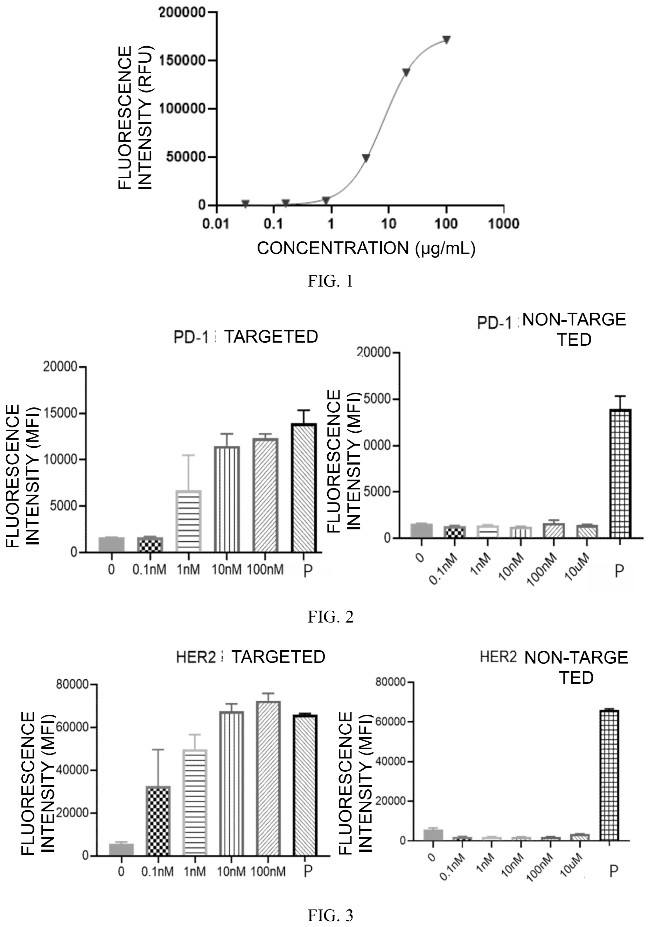

As illustrated in FIG. 1, the enzymatic activity of NEU2-M-anFcVH was close to the upper limit of detection at 100 µg/mL, and the EC₅₀ was about 8.0 µg/mL, indicating high enzymatic activity.

### Example 3: Affinity of Sialidase NEU2-M-anFcVH Fusion Protein (SEQ ID NO. 29) to IgG1 and IgG4

The affinity of NEU2-M-anFcVH to IgG1 and IgG4 was determined using Fortebio. Since NEU2-M-anFcVH has a his-tag, 20 µg/mL of NEU2-M-anFcVH protein was loaded using the HIS1K probe at a loading height of 0.5 nm. Antibody-1 (anti-Her2 antibody, the amino acid sequences of the light and heavy chains are shown in SEQ ID NOs. 1 to 3) and Keytruda (anti-PD-1 antibody) are antibodies of the IgG1 and IgG4 types, respectively. Starting from 1500 nM, Antibody-1 and Keytruda were serially diluted 2-fold for 5 times, and the qualified data were selected for affinity calculation.

**Table 2 Affinity of NEU2-M-anFcVH to IgG1 and IgG4**

| **Sample name** | **KD (M)** | **kₐ (1/Ms)** | **k_{dis} (1/s)** |
|---|---|---|---|
| Antibody-1 | 3.99E-08 | 7.27E+03 | 2.90E-04 |
| Keytruda | 3.17E-08 | 6.57E+03 | 2.08E-04 |

As illustrated in Table 2, NEU2-M-anFcVH bound to both antibody-1 and Keytruda, with an affinity of 10⁻⁸ M, which was sufficient to bind to a targeting antibody and form a complex.

### Example 4: Detection of Enzymatic Cleavage Activity of Complex for Sialic Acid on Surface of Target Cells In Vitro

SKBR3 and Jurkat T cells in the logarithmic growth phase were collected along with the complete medium, counted, and centrifuged at 300 g for 5 minutes. The cells were then resuspended in the corresponding serum-free medium and adjusted to a density of 2×10⁶ cells/ml. A 100 µL aliquot of the cell suspension was added to each well of a flat-bottom 96-well plate.. 100 µL of different concentrations of targeting protein (antibody) complexes (NEU2-M-anFcVH with antibody-1 and NEU2-M-anFcVH with Keytruda (purchased from Merck) were used as targeting complexes for HER2 and PD-1, respectively, non-targeting IgG1 antibody formed a non-targeting complex as negative control, bacterial sialidase α2-3, 6, 8, 9, Neuraminidase (purchased from RhinoBio) were used as positive controls P. These complexes were incubated with cells in a 37°C incubator for 1 hour. After mixing with each well tip, 100 µL was added to a new 96-well plate, centrifuged at 300 g for 5 minutes, and washed twice with PBS. The cells were resuspended with 100 µL of PNA-AF647 reagent or blocking buffer, incubated at 4°C for 30 minutes in the dark, centrifuged at 300 g for 5 minutes, and washed twice with blocking buffer. The cells were then resuspended in 200 µL of blocking buffer and analyzed.

PNA staining was used to show the effect of Neu2 cleavage of sialic acid on galactose exposure. The effect of Neu2 binding on sialic acid cleavage on target cells was detected by selecting SK-BR-3 (HER2+++) and Jurkat T (PD-1 effector) cells (see FIGS. 2 and 3). This demonstrated that, at the same low concentration of sialidase, the targeting protein (antibody) complexes had a very clear ability to degrade sialic acid on the surface of target cells, while the non-targeting antibody complex could not effectively degrade sialidase on the surface of target cells.

### Example 5: Enzymatic Activity Detection in Sialidase-Z33 Fusion Protein

Based on the quantitative results, the supernatant of the fusion protein (hNEU2-M-Z33 or Z33-hNEU2-M) was diluted with PBS, using PBS as a blank control. The enzyme cleavage substrate 4-MU-NANA was diluted to 2 mM and mixed with hNEU2-M-Z33 or Z33-hNEU2-M at a 1:1 volume ratio. After incubation at 37°C for 2 hours, the reaction was terminated by adding 0.1 M Na₂CO₃ at pH 10.5. The fluorescence values of λₑₓ at 365 nm and λₑₘ at 450 nm were detected using a microplate reader. The higher the fluorescence value, the higher the enzymatic activity.

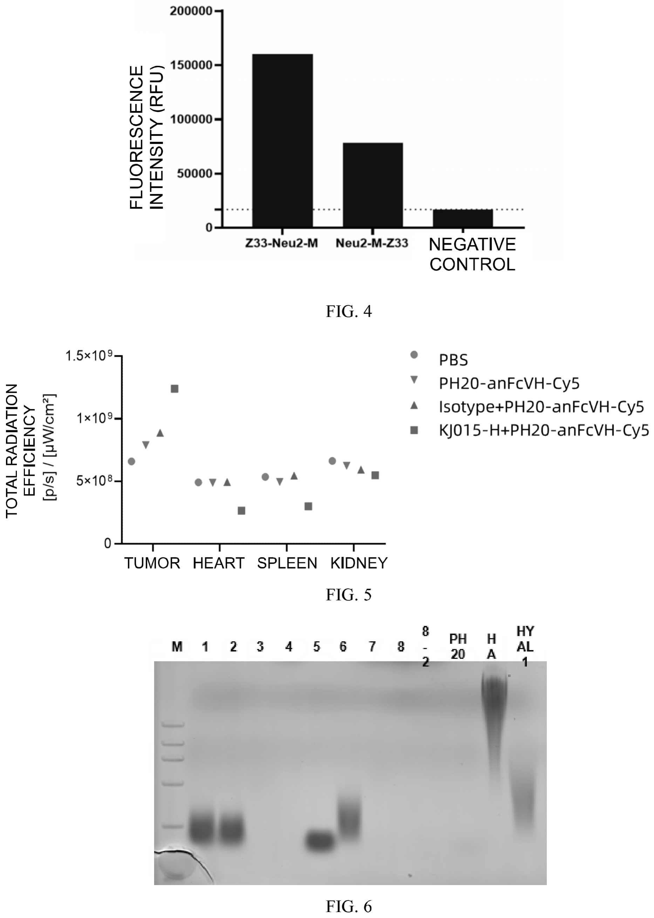

The results are illustrated in FIG. 4. The supernatants of both proteins exhibited sufficient enzymatic activity.

### Example 6: Confirmation of Affinity between Fusion Protein and Fc using Fortebio

The affinity between the fusion protein and Fc was detected using Fortebio. Two types of supernatant were loaded with the HIS1K probe, and the load response values were set to 0.5 nm to 0.8 nm. Different concentrations of antibody were then bound. Starting from 183.5 nM, the antibody (antibody-1) was serially diluted 2-fold for 5 times, and the qualified data were selected for affinity calculation.

**Table 3 Affinity of fusion protein to Fc**

| Molecular name | Affinity to IgG | | |
|---|---|---|---|
| | KD (M) | Kₐ (1/Ms) | K_{dis} (1/s) |
| hNEU2-M-Z33 | 1.73E-09 | 2.99E+05 | 5.15E-04 |
| Z33-hNEU2-M | 6.72E-10 | 4.30E+05 | 2.89E-04 |
| Z33* | 4.2E-08 | 4.6E+05 | 2E-02 |

| | | | |
|---|---|---|---|
| Note: * indicates literature data | | | |

The results are shown in Table 3. Z33 can still bind to the antibody after fusing with hNEU2-M. Compared with the Z33 molecule alone, the fusion protein exhibited higher affinity to the antibody, reaching the pM level.

### Example 7: Affinity Detection

The affinity between the Z33 mutant and antibody-1 was determined using bio-layer interferometry (BLI). The experiments were performed on a Fortebio Octet Red384 instrument, following the standard operating procedure manual. All samples were stored in PBS containing 0.1% BSA and 0.05% Tween (PBST BSA). Compared with wild-type sequences, detailed data on the relevant sequences are shown in Table 4. Sequences exhibiting increased affinity were SEQ ID NO: 34, SEQ ID NO: 36, SEQ ID NO: 38, and SEQ ID NO: 39, namely the single mutation Z33 Q4L or Z33 S29Q, and the double mutations Z33 Q4L-S29Q and Z33 Q4L-S29K. Binding was not detected in the double mutants Z33 Q6W-S29K and Z33 F1L-Q6W.

**Table 4 Detection of affinity between Z33 mutants and antibody-1**

| Mutation | Expression sequence SEQ ID NO: | KD (M) | Kₐ (1/Ms) | K_{dis} (1/s) |
|---|---|---|---|---|
| Z33 WT | 18 | 2.68E-09 | 3.35E+05 | 8.96E-04 |
| Z33 Q4L S29K | 34 | 1.54E-09 | 2.85E+05 | 4.39E-04 |
| Z33 F1L Q4L | 35 | 3.70E-09 | 3.27E+05 | 1.21E-03 |
| Z33 Q4L S29Q | 36 | 2.14E-09 | 2.97E+05 | 6.35E-04 |
| Z33 F1L S29Q | 37 | 3.55E-09 | 3.49E+05 | 1.24E-03 |
| Z33 Q4L | 38 | 2.03E-09 | 3.16E+05 | 6.41E-04 |
| Z33 S29Q | 39 | 1.64E-09 | 3.32E+05 | 5.44E-04 |
| Z33 Q6W S29K | 40 | N/A | N/A | N/A |
| Z33 F1L Q6W | 41 | N/A | N/A | N/A |

The fusion protein obtained by fusing the Z33 mutants with LIGAND A also exhibited excellent affinity. Compared with the affinity between wild-type Z33 in Table 5 and antibody-1, the fusion proteins of the Z33 Q4L mutant and Z33 S29Q mutant showed significantly increased affinity to antibody-1. The affinity of the Z33 F1L mutant was lower than that of the wild-type. In addition, mutations in Z33 S29R/K and Q4L significantly mitigated the affinity decrease caused by mutations at other sites in Z33.

**Table 5 Detection of affinity between Z33 mutant fusion proteins and IgG**

| Molecular name | Affinity to IgG | | |
|---|---|---|---|
| | KD (M) | Kₐ (1/Ms) | K_{dis} (1/s) |
| WT | 2.25E-09 | 2.72E+05 | 1.46E-03 |
| LIGAND A-Z33 Q4L | 1.19E-09 | 2.82E+05 | 3.36E-04 |
| LIGAND A-Z33 S29Q | 1.21E-09 | 2.78E+05 | 3.36E-04 |
| LIGAND A-Z33 P16A-N17L-N18M | 2.51E-08 | 3.02E+05 | 7.58E-03 |
| LIGAND A-Z33 P16A-N17L-N18M-S29R | 1.23E-08 | 2.44E+05 | 3.00E-03 |
| LIGAND A-Z33 P16A-N17L-N18M-S29N | 8.40E-08 | 2.73E+05 | 2.29E-02 |
| LIGAND A-Z33 A12T-H14W-P16L-N17T | 3.19E-08 | 3.40E+05 | 1.08E-02 |
| LIGAND A-Z33 A12T-H14W-P16L-N17T-S29K | 1.47E-08 | 2.95E+05 | 4.33E-03 |
| LIGAND A-Z33 E11D-P16L-N17L | 2.17E-08 | 2.36E+05 | 5.13E-03 |
| LIGAND A-Z33 Q4L-E11D-P16L-N17L | 1.55E-08 | 2.71E+05 | 4.21E-03 |
| LIGAND A-Z33 F1L | 6.19E-09 | 2.67E+05 | 1.65E-03 |
| LIGAND A-Z33 Q6W | N/A | N/A | N/A |

These results demonstrated that the fusion proteins constructed from Z33 and mutants thereof also possess the potential for purification and detection of Fc-containing molecules, suitable for large-scale production of such compounds, or as Fc engineering tools for drug design and development.

### Confirmation of Affinity between Fusion Protein and Fc using Fortebio

Fortebio was used to detect the affinity between the fusion protein and atezolizumab. Four proteins were loaded with the HIS1K probe, and the load response values were set to 0.5 nm. Different concentrations of antibody were then bound. Starting from 100 nM, the antibody (atezolizumab) was serially diluted 2-fold for 6 times, and appropriate data were selected for affinity calculation.

**Table 6 Affinity of fusion protein to Fc**

| Molecular name | Z33 mutation site | Affinity to IgG KD (M) |
|---|---|---|
| PH20-Z33-mut1 | S29Q | 1.54E-09 |
| Z33-mut1-PH20 | S29Q | 3.91E-11 |
| PH20-Z33-mut2 | Q4L | 1.21E-09 |
| Z33-mut2-PH20 | Q4L | 3.47E-10 |

### Example 8: Immunogenicity Prediction

The MHC I immunogenicity of each peptide sequence was predicted using the publicly available bioinformatics tool NetMHCpan-4.1, and the MHC II immunogenicity was predicted using NetMHCIIpan-4.0 (https://doi.org/10.1093/nar/gkaa379). SEQ ID NOs: 36, 37, 38, and 39 showed decreased MHC I and II immunogenicity scores compared with SEQ ID NO: 18.

### Example 9: Detection of Targeting Binding Effect of PH20-anFcVH and Antibody-1 Targeting Complex on HER2+ Tumor Cells in Tumor-Bearing Mice

The purified PH20-anFcVH fusion protein and the anti-HER2 antibody antibody-1 were mixed to form a targeting complex.

Protein labeling with fluorescence: 1 mg of PH20-anFcVH fusion protein (2.45 mg/mL) sample was transferred to a 1.5 mL Eppendorf tube. Cy5-nhs solution at a concentration of 5 mg/mL was added according to a dye labeling molar ratio of 10/1 (dye/labeled sample). Simultaneously, 10 µL of triethylamine was added, and the mixture was incubated overnight at room temperature on a 3D shaker.

Fluorescent protein purification:
a. A spin column was placed in a 2 mL Eppendorf tube. The purification resin was mixed to ensure a homogeneous suspension. 250 µL of suspension was added to the spin column, and the stopper was removed. The column was centrifuged at 1000 g for 2 minutes to remove the stock solution, and 250 µL of PBS was added, and the column was centrifuged again at 1000 g for 2 minutes for washing. The used collection tube was discarded, and a new collection tube with a column was placed.
b. 250 µL of labeled solution was added to the spin column, and the sample was mixed with the resin by a brief vortex. The spin column was centrifuged at 1000 g for 2 minutes, purified samples were collected, and the same purified samples were combined. The PH20-anFcVH fusion protein-Cy5 molecule was obtained.

Human breast cancer HCC1569 cell line was subcutaneously xenografted into NOD/SCID female mice. Tumor-bearing mice with a tumor volume of 200 mm³ were injected intravenously with 100 µL of the drug via the tail vein (Gl: PBS, G2: PH20-anFcVH fusion protein-Cy5, G3: Isotype-IgG + PH20-anFcVH-Cy5, G4: antibody-1 + PH20-anFcVH-Cy5). The dose of PH20-anFcVH-Cy5 was the same in all groups. 54.5 hours after administration, tumors, hearts, spleens, and kidneys were collected, placed on black paper, and the Cy5 channel (649/666) was detected by fluorescence imaging.

The results are shown in FIG. 5. Group G4 (antibody-1 + PH20-anFcVH-Cy5 fluorescence) showed better targeting effect on hHER2-HCC1569 tumors than groups G2 and G3. The targeting complex binds to HER2 molecules on the surface of tumor cells, while binding is less in other organs where HER2 molecules are not positive, which reflects the enrichment effect of the complex on the surface of cells where the target molecule is located.

### Example 10: Detection of Affinity between Fc and Different Hyaluronidases after fusion with VHH

The affinity between the fusion protein and Fc was detected using Fortebio. The expression supernatant of four hyaluronidase-VHH fusion protein molecules (Table 7, numbers 1 to 4) were loaded with the HIS1K probe, and the load response value was set to 0.5 nm to 0.8 nm. Different concentrations of antibody were then bound. Starting from 1000 nM, the antibody was serially diluted 2-fold for 4 times, and affinity was calculated.

**Table 7 Affinity results of four hyaluronidase-VHH fusion protein molecules with Fc**

| Number | Molecular name | Affinity to IgG | | |
|---|---|---|---|---|
| | | KD (M) | Kₐ (1/Ms) | K_{dis} (1/s) |
| 1 | HYAL1-anFcVH | 4.95E-08 | 5.62E+03 | 2.78E-04 |
| 2 | PH20-anFcVH | 1.01E-08 | 7.76E+03 | 7.86E-05 |
| 3 | anFcVH-HYAL1 | 5.17E-12 | 9.33E+03 | <1.0E-07 |
| 4 | anFcVH-PH20 | 7.41E-08 | 9.71E+03 | 7.19E-04 |
| 5 | anFcVH | 6.10E-12 | 8.48E+03 | <1.0E-07 |

The results are shown in Table 7, and all fusion proteins bind strongly to antibodies.

### Example 11: Detection of Enzymatic Activity in Hyaluronidase-VHH Fusion Protein

Four expression supernatants were diluted 50-fold with enzyme diluent and mixed 1:1 with a 0.5 mg/mL high molecular sodium hyaluronate solution, then incubated at 37°C for 1 hour. A 0.5% TAE agarose gel was prepared, and the resulting mixture was subjected to agarose gel electrophoresis at 50 V for 2 to 3 hours. A 50% ethanol staining solution containing 0.005% Stains-All was prepared, and staining was performed overnight in the dark after electrophoresis. The stain was then destained under purified water for 1 hour, followed by scanning and imaging.

The results are shown in FIG. 6. All four proteins showed enzymatic activity after their supernatants were diluted 100-fold. The PH20 fusion proteins (lanes 3 and 7 in FIG. 6, corresponding to molecules 2 and 4 in Table 7) could completely degrade the substrate sodium hyaluronate, consistent with the properties of PH20 alone. The HYAL1 fusion protein (lanes 1 and 5 in FIG. 6, corresponding to molecules 1 and 3 in Table 7) has the same properties as HYAL1 alone, both of which can degrade large molecules of sodium hyaluronate into smaller molecules.

### Example 12: Detection of Efficacy of Targeting Complex Keytruda + NEU2-M-anFcVH in Mouse Model

To investigate the efficacy of the targeting complex in the BALB/c-hPD1/hPDL1 model, the administration methods and study groups were set up as shown in Table 8. Six mice were included in each group, and the drugs were administered twice a week for three weeks. This study aimed to investigate the antitumor effect of the targeting complex.

**Table 8 Efficacy of each administration method of Keytruda + Neu2-M-anFcVH in CT26-hPDL1 model**

| Group | Sample type | Sample | Number of animals | Administration method | Dose |
|---|---|---|---|---|---|
| G1 | Blank control | Normal saline | 6 | *i.p.* | / |
| G2 | Single drug control | Keytruda | 6 | *i.p.* | 12 mg/kg |
| G3 | | Neu2-M-anFcVH | 6 | *i.p.* | 10 mg/kg |
| G4 | Test sample | Keytruda + NEU2-M-anFcVH | 6 | *i.p.* | 12 mg/kg |
| | | | | | **+ 2.5** mg/kg |

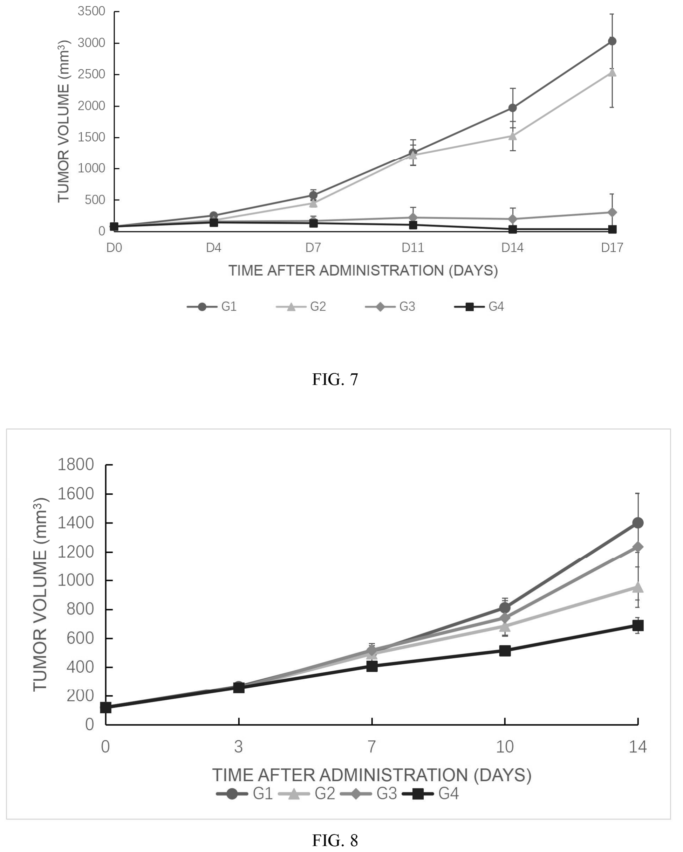

The results (FIG. 7) showed that Keytruda + NEU2-M-anFcVH exhibited good antitumor activity, which was significantly better than Keytruda.

### Example 13: Detection of Efficacy of Targeting Complex Atezolizumab + Z33-mutl-PH20 in PBMC NCI-H292 Model

To investigate the efficacy of the targeting complex in the PBMC NCI-H292 model, the administration methods and study groups were set up as shown in Table 9. Six NOG mice were included in each group, and the drugs were administered twice a week for two consecutive weeks. Tumor growth was then monitored.

**Table 9 Efficacy of each administration method of Atezolizumab + Z33-mutl-PH20 in PBMC NCI-H292 model**

| **Group** | **Sample type** | **Sample** | **Number of animals** | **Administration method** | **Dose** |
|---|---|---|---|---|---|
| G1 | Blank control | Normal saline | 6 | *i.v.* | / |
| G2 | Negative control | Atezolizum ab | 6 | *i.v.* | 10 mg/kg |
| G3 | | Z33-mut1-P H20 | 6 | *i.v.* | 0.04 mg/kg |
| G4 | Test sample | Atezolizum ab + Z33-mut1-P H20 | 6 | *S. C.* | 10 mg/kg + 0.01 mg/kg |

The results (FIG. 8) showed that Atezolizumab + Z33-mut1-PH20 exhibited good antitumor activity, which was significantly better than Atezolizumab.

### Example 14: Detection of Efficacy of Targeting Complex Antibody-1 + NEU2-M-anFcVH in EMT6-HER2 Model

To investigate the efficacy of the targeting complex in the EMT6-hHER2 model, the administration methods and study groups were set up as shown in Table 10. Six mice were included in each group, and the drugs were administered twice a week for three weeks. This study aimed to investigate the antitumor effect of the targeting complex.

**Table 10 Efficacy of each administration method of antibody-1 + NEU2-M- anFcVH in EMT6-hHER2 model**

| **Group** | **Sample type** | **Sample** | **Number of animals** | **Administration method** | **Dose** |
|---|---|---|---|---|---|
| G1 | Blank control | Normal saline | 6 | *i.p.* | / |
| G2 | Negative control | Antibody-1 | 6 | *i.p.* | 10 mg/kg |
| G3 | | NEU2-M-anFcVH | 6 | *i.p.* | 4 mg/kg |
| G4 | Test sample | Antibody-1 + NEU2-M-anFcVH | 6 | *i.p.* | 10 mg/kg +4 mg/kg |
| G5 | Test sample (low dose) | Antibody-1 + NEU2-M-anFcVH | 6 | *i.p.* | 10 mg/kg + 0.4 mg/kg |

### Example 15: Detection of Efficacy of Targeting Complex Antibody-1 + PH20-anFcVH in EMT6-hHER2 Model

To investigate the efficacy of the targeting complex in the EMT6-hHER2 model, the administration methods and study groups were set up as shown in Table 11. Six mice were included in each group, and the drugs were administered twice a week for three weeks.

**Table 11 Efficacy of each administration method of antibody-1 + PH20-anFcVH in EMT6-hHER2 model**

| **Group** | **Sample type** | **Sample** | **Number of animals** | **Administration method** | **Dose** |
|---|---|---|---|---|---|
| G1 | Blank control | Normal saline | 6 | *i.p.* | / |
| G2 | Negative control | Antibody-1 | 6 | *i.p.* | 10 mg/kg |
| G3 | Test sample | Antibody-1 + PH20-anFc VH | 6 | *i.v.* | 10 mg/kg + 0.45 mg/kg |

Although specific embodiments of the present disclosure have been described above, those skilled in the art should understand that these embodiments are merely illustrative examples, and various changes or modifications can be made to these embodiments without departing from the principles and essence of the present disclosure. Therefore, the scope of protection of the present disclosure is defined by the appended claims.

## Claims

1. A targeting complex, comprising:
a first molecule comprising an Fc fragment moiety and a target-binding fragment moiety; and
a second molecule comprising a first moiety that is an enzyme and a second moiety capable of non-covalently binding to the first molecule;
optionally, the first molecule is a targeting protein comprising an Fc fragment moiety and a target-binding fragment moiety, and the second molecule is a fusion protein.

2. The targeting complex according to claim 1, wherein
the first molecule comprises an antibody or a fusion protein, each of which comprises an Fc fragment, and optionally, the antibody is selected from an anti-Her2 antibody, an anti-PD-1 antibody, or an anti-PD-L1 antibody.

3. The targeting complex according to claim 1 or 2, wherein
the non-covalent binding is a protein-protein interaction, and optionally, the protein-protein interaction is selected from antigen-antibody binding, Protein A/Protein G-antibody Fc binding, or antibody Fc receptor-antibody Fc binding.

4. The targeting complex according to any one of claims 1 to 3, wherein
the second molecule retains enzymatic activity and/or the first molecule retains targeting activity, and optionally, after the targeting complex binds to a target molecule, the second molecule is capable of acting on a substrate molecule of the enzyme around the target molecule.

5. The targeting complex according to any one of claims 1 to 4, wherein
the enzyme is an extracellular molecule-modifying enzyme,
optionally, the enzyme is selected from a hyaluronidase, a sialidase, an N-glycosidase, an O-glycosidase, a collagenase, a heparinase, a chondrosulphatase, or a chondroitinase,
optionally, the sialidase is selected from *Salmonella typhimurium* sialidase, *Vibrio cholerae* sialidase, human neuraminidase 1, human neuraminidase 2, human neuraminidase 3, or human neuraminidase 4,
optionally, the N-glycosidase is selected from Endo H, PNGase F, PNGase A, Endo S, Endo S2, Endo D, Endo F2, or Endo F3,
optionally, the O-glycosidase is selected from Endo-O-Glycosidase or Exo-O-Glycosidase,
optionally, the hyaluronidase is selected from HYAL1, HYAL2, HYAL3, HYAL4, HYALP1, or PH20/SPAM1,
optionally, the chondroitinase is selected from chondroitinase C, chondroitinase B, hyaluronidase ACIII, chondroitinase ACII, chondroitinase AC, or chondroitinase ABC,
optionally, the heparinase is selected from heparinase I, heparinase II, or heparinase III, and/or
optionally, the collagenase is selected from collagenase 1, collagenase 2, collagenase 3, or serine protease Cathepsin L.

6. The targeting complex according to any one of claims 1 to 5, wherein
the second moiety of the second molecule is capable of non-covalently binding to a constant region of the first molecule,
optionally, the second moiety of the second molecule is capable of non-covalently binding to the Fc fragment moiety of the first molecule,
optionally, an affinity, expressed as KD, of the second moiety of the second molecule to the Fc fragment moiety of the first molecule is 1 × 10⁻⁷ M to 1 × 10⁻¹² M,
optionally, the second moiety of the second molecule is selected from an antibody or an antigen-binding fragment thereof (for example, VHH, affibody, scFv, Fab, or F(ab')2) capable of binding to a constant region fragment of an antibody, or selected from Protein A or an Fc-binding fragment thereof, affibody or an Fc-binding fragment thereof, Protein G or an Fc-binding fragment thereof, or Fc receptor or an Fc-binding fragment thereof capable of binding to an Fc region of an antibody, or is an Fc-binding peptide,
optionally, the enzyme and the second moiety of the second molecule are connected via a peptide bond or a linker, and optionally, the linker is a peptide linker.

7. The targeting complex according to any one of claims 1 to 6, wherein
the second moiety of the second molecule is a wild-type Z33 or a polypeptide having an amino acid mutation relative to the wild-type Z33.

8. The targeting complex according to claim 7, wherein
a position of the amino acid mutation includes at least one of positions 1, 4 and 29, and the wild-type Z33 has an amino acid sequence shown in SEQ ID NO: 1.

9. The targeting complex according to claim 7 or 8, wherein
the polypeptide has an amino acid substitution relative to the wild-type Z33,
optionally, the substituted amino acid at the position 1 is selected from F or L,
optionally, the substituted amino acid at the position 4 is selected from Q, F, E, R, or L, and
optionally, the substituted amino acid at the position 29 is selected from S, Q, R, or K.

10. The targeting complex according to any one of claims 7 to 9, wherein
the amino acid sequence of the polypeptide has at least 50%, at least 60%, at least 70%, at least 80%, at least 85%, at least 90%, at least 92%, at least 95%, or at least 97% identity to the amino acid sequence shown in SEQ ID NO: 1.

11. The targeting complex according to any one of claims 7 to 10, wherein
the polypeptide includes the following amino acid sequence: 
X1X2X3X4X5X6X7X8X9X10X11X12X13X14X15X16X17X18X19X20X21X22X23X24X25X26X
27X28X29X30X31X32X33,
 where
X₁ is selected from F or L,
X₄ is selected from Q, F, E, R, or L,
X₂₉ is selected from S, Q, R, or K, further
X₂ is selected from N, E, or S,
X₃ is selected from M, K, or E,
X₅ is selected from Q or C,
X₆ is selected from Q, W, L, K, or A,
X₇ is selected from R, L, K, N, A, D, or C,
X₈ is selected from R or A,
X₉ is selected from F, M, or A,
X₁₀ is selected from Y, A, or S,
X₁₁ is selected from E, A, D, or S,
X₁₂ is selected from A, I, T, or E,
X₁₃ is selected from L, A, G, or K,
X₁₄ is selected from H, A, V, F, W, or G,
X₁₅ is selected from D, V, C, N, or L,
X₁₆ is selected from P, L, or A,
X₁₇ is selected from N, L, T, or G,
X₁₈ is selected from L, M, or V,
X₁₉ is selected from N, G, or T,
X₂₀ is selected from E, K, A, or Q,
X₂₁ is selected from E or Q,
X₂₂ is Q,
X₂₃ is R,
X₂₄ is selected from N, W, A, or K,
X₂₅ is selected from A or G,
X₂₆ is selected from K, R, or F,
X₂₇ is I,
X₂₈ is selected from K, C, Q, or R,
X₃₀ is selected from I, F, or L,
X₃₁ is selected from R, M, or K,
X₃₂ is selected from D, N, K, or E, and
X₃₃ is selected from D, N, or E.

12. The targeting complex according to claim 11, wherein
X₇ is R,
X₉ is F,
X₁₀ is Y,
X₁₃ is L,
X₂₃ is R, and/or
X₃₀ is I.

13. The targeting complex according to claim 11 or 12, wherein
X₁₁ is E or D,
X₁₂ is A or T,
X₁₄ is H or W,
X₁₆ is P, A or L,
X₁₇ is N, L or T,
X₁₈ is N or M, and/or
X₂₃ is R.

14. The targeting complex according to any one of claims 11 to 13, wherein
X₁ is L,
X₄ is L, and/or
X₂₉ is Q, K, or R.

15. The targeting complex according to claim 5, wherein
the hyaluronidase has an amino acid sequence shown in any one of SEQ ID NOs. 6, 7, or 65 to 71, the sialidase has an amino acid sequence shown in any one of SEQ ID NOs. 4, 5, or 55 to 60, the collagenase has an amino acid sequence shown in SEQ ID NO. 8, the heparinase has an amino acid sequence shown in SEQ ID NO. 9, the chondrosulphatase has an amino acid sequence shown in SEQ ID NO. 10, and the chondroitinase has an amino acid sequence shown in SEQ ID NO. 11.

16. The targeting complex according to any one of claims 1 to 15, wherein
the second moiety of the second molecule is an antibody or an antigen-binding fragment thereof including CDR1 to CDR3, and amino acid sequences of CDR1 to CDR3 respectively include SEQ ID NOs. 31 to 33 according to the Kabat numbering scheme,
optionally, the second moiety of the second molecule includes a polypeptide having an amino acid sequence shown in any one of SEQ ID NOs. 12 to 18 and SEQ ID NOs. 34 to 39, or an Fc-binding fragment thereof,
optionally, the second moiety of the second molecule includes a polypeptide having an amino acid sequence selected from any one of SEQ ID NOs. 34 to 39, or an Fc-binding fragment thereof, and
optionally, the second moiety of the second molecule has an amino acid sequence shown in SEQ ID NO. 34, 36, 38, or 39.

17. The targeting complex according to any one of claims 1 to 16, wherein
the fusion protein serving as the second molecule is selected from a polypeptide having an amino acid sequence shown in any one of SEQ ID NOs. 19 to 30 or SEQ ID NOs. 61 to 64.

18. A molecule comprising: a first moiety that is an enzyme; and a second moiety capable of non-covalently binding to a constant region of an antibody, optionally, the molecule being a fusion protein.

19. The molecule according to claim 18, wherein
the non-covalent binding is a protein-protein interaction, and optionally, the protein-protein interaction is selected from antigen-antibody binding, Protein A/Protein G-antibody Fc binding, or antibody Fc receptor-antibody Fc binding, and
optionally, an affinity, expressed as KD, of the second moiety to the constant region of the antibody is 1 × 10⁻⁷ M to 1 × 10⁻¹² M.

20. The molecule according to claim 18 or 19, wherein
the molecule retains enzymatic activity,
optionally, after binding to a first molecule, the molecule is capable of modifying a substrate molecule of the enzyme around a target of the first molecule, and the first molecule includes an Fc fragment moiety and a target-binding fragment moiety, and
optionally, the first molecule includes an antibody or a fusion protein including an Fc fragment.

21. The molecule according to any one of claims 18 to 20, wherein
the enzyme is an extracellular molecule-modifying enzyme,
optionally, the enzyme is selected from a hyaluronidase, a sialidase, an N-glycosidase, an O-glycosidase, a collagenase, a heparinase, a chondrosulphatase, or a chondroitinase,
optionally, the sialidase is selected from Salmonella typhimurium sialidase, Vibrio cholerae sialidase, human neuraminidase 1, human neuraminidase 2, human neuraminidase 3, or human neuraminidase 4,
optionally, the N-glycosidase is selected from Endo H, PNGase F, PNGase A, Endo S, Endo S2, Endo D, Endo F2, or Endo F3,
optionally, the O-glycosidase is selected from Endo-O-Glycosidase or Exo-O-Glycosidase,
optionally, the hyaluronidase is selected from HYAL1, HYAL2, HYAL3, HYAL4, HYALP1, or PH20/SPAM1,
optionally, the chondroitinase is selected from chondroitinase C, chondroitinase B, hyaluronidase ACIII, chondroitinase ACII, chondroitinase AC, or chondroitinase ABC,
optionally, the heparinase is selected from heparinase I, heparinase II, or heparinase III, and/or
optionally, the collagenase is selected from collagenase 1, collagenase 2, collagenase 3, or serine protease Cathepsin L.

22. The molecule according to any one of claims 18 to 21, wherein
the second moiety of the molecule is capable of non-covalently binding to a constant region of the first molecule,
optionally, the second moiety of the molecule is capable of non-covalently binding to an Fc region of the first molecule, and
optionally, the second moiety of the molecule is selected from an antibody or an antigen-binding fragment thereof capable of binding to a constant region fragment of an antibody (for example, VHH, scFv, Fab or F(ab')2), or selected from Protein A or an Fc-binding fragment thereof, Protein G or an Fc-binding fragment thereof, or Fc receptor or an Fc-binding fragment thereof capable of binding to an Fc region of an antibody, or is an Fc-binding peptide.

23. The molecule according to any one of claims 18 to 22, wherein
the second moiety of the molecule is the second moiety of the second molecule in the targeting complex according to any one of claims 7 to 14.

24. The molecule according to claim 21, wherein
the hyaluronidase has an amino acid sequence shown in any one of SEQ ID NOs. 6, 7, or 65 to 71,
the sialidase has an amino acid sequence shown in any one of SEQ ID NOs. 4, 5, or 55 to 60,
the collagenase has an amino acid sequence shown in SEQ ID NO. 8,
the heparinase has an amino acid sequence shown in SEQ ID NO. 9,
the chondrosulphatase has an amino acid sequence shown in SEQ ID NO. 10, and
the chondroitinase has an amino acid sequence shown in SEQ ID NO. 11.

25. The molecule according to any one of claims 18 to 24, wherein
the second moiety of the molecule is an antibody or an antigen-binding fragment thereof including heavy chain variable regions CDR1 to CDR3, and amino acid sequences of the heavy chain variable regions CDR1 to CDR3 respectively include SEQ ID NOs. 31 to 33 according to the Kabat numbering scheme,
optionally, the second moiety of the molecule includes a polypeptide having the amino acid sequence shown in any one selected from SEQ ID NOs. 12 to 18 and SEQ ID NOs. 34 to 39, or an Fc-binding fragment thereof,
optionally, the second moiety of the second molecule is selected from a polypeptide having the amino acid sequence shown in any one of SEQ ID NOs. 34 to 39, or an Fc-binding fragment thereof, and
optionally, the second moiety of the second molecule has an amino acid sequence shown in SEQ ID NO. 34, 36, 38, or 39.

26. The molecule according to any one of claims 18 to 25, wherein
the molecule is selected from a polypeptide having an amino acid sequence shown in any one of SEQ ID NOs. 19 to 30 or SEQ ID NOs. 61 to 64.

27. A polypeptide, wherein
the polypeptide is the second moiety of the second molecule in the targeting complex according to any one of claims 7 to 14, the second moiety of the second molecule being the polypeptide having an amino acid mutation relative to the wild-type Z33,
optionally, the polypeptide having the amino acid mutation relative to the wild-type Z33 has an amino acid sequence selected from any one of SEQ ID NOs. 34 to 39,
optionally, the polypeptide having the amino acid mutation relative to the wild-type Z33 has an amino acid sequence selected from any one of SEQ ID NOs. 34 to 39, and
optionally, the amino acid sequence of the polypeptide having the amino acid mutation relative to the wild-type Z33 has an amino acid sequence shown in SEQ ID NO. 34, 36, 38, or 39.

28. A composition comprising:
the targeting complex according to any one of claims 1 to 17, the molecule according to any one of claims 18 to 26, or the polypeptide according to claim 27; and
a pharmaceutically acceptable carrier.

29. A nucleic acid encoding the targeting complex according to any one of claims 1 to 17, the molecule according to any one of claims 18 to 26, or the polypeptide according to claim 27.

30. An expression vector comprising the nucleic acid according to claim 29.

31. A host cell comprising the nucleic acid according to claim 29 or the expression vector according to claim 30.

32. The host cell according to claim 31, wherein
the host cell is a mammalian host cell, a prokaryotic host cell, or a yeast host cell.

33. A method comprising:
administering, to an individual in need thereof, the targeting complex according to any one of claims 1 to 17, the molecule according to any one of claims 18 to 26, or the composition according to claim 28.

34. A method for treating cancer, the method comprising:
administering, to an individual suffering from the cancer, the targeting complex according to any one of claims 1 to 17, the molecule according to any one of claims 18 to 26, or the composition according to claim 28,
wherein, optionally, the cancer is breast cancer.

35. A use of the polypeptide according to claim 27, wherein
the use is selected from:
(a) detecting an Fc-containing molecule, or
(b) purifying the Fc-containing molecule,
wherein, optionally, the Fc-containing molecule is IgG.
